# EUROPEAN PATENT APPLICATION

(11) **EP 4 524 565 A1**
(43) Date of publication of application: **19.03.2025**
(21) Application number: 24199912.7
(22) Date of filing: 12.09.2024
(51) Int. Cl.: G01N 33/542, G01N 21/64, G01N 33/58, G01N 33/68, C07K 7/08, C07K 7/06, C07K 5/11

(54) **METHODS FOR MEASURING INTERACTIONS BETWEEN PROTEINS COMPRISING AN FC PORTION AND PARTICLES**

(30) Priority: 12.09.2023 EP 23196764
(71) Applicant: NanoTemper Technologies GmbH, 81369 München (DE)
(72) Inventor: HEIM, Christopher, 81369 München (DE); STREICHER, Werner, 81369 München (DE); BAASKE, Philipp, 81369 München (DE); SCHRAMM, Philipp, 81369 München (DE)
(74) Representative: Schiweck Weinzierl Koch Patentanwälte Partnerschaft mbB

(57) **Abstract**

The present invention relates to means and methods for for measuring an interaction between a fluorescently labelled protein comprising an Fc portion and particles in a solution, wherein said protein comprising an Fc portion is fluorescently labelled by a Fc-binding peptide comprising a fluorescent label. Means may, e.g. be kits or systems which may also be used measuring an interaction between a fluorescently labelled protein comprising an Fc portion and particles in a solution.

## Description

The present invention relates to methods for measuring an interaction between a fluorescently labelled protein comprising an Fc portion and particles in a solution, wherein said protein comprising an Fc portion is fluorescently labelled by a Fc-binding peptide comprising a fluorescent label. The present invention further relates to the use of a kit for performing the method for measuring an interaction between a fluorescently labelled protein comprising an Fc portion and particles in a solution comprising a fluorescent label ready to be coupled to a Fc-binding peptide and a Fc-binding peptide. The present invention further relates to a kit for use in the method for measuring an interaction between a fluorescently labelled protein comprising an Fc portion and particles in a solution, comprising a fluorescent label ready to be coupled to a Fc-binding peptide and a Fc-binding peptide. The present invention further relates to a system comprising an Fc-binding peptide comprising a fluorescent label, a protein comprising an Fc portion which is to be bound by an Fc-binding peptide and a device configured to analyse alterations in the fluorescence spectrum of the labelled protein comprising an Fc portion upon a potential interaction of the labelled protein comprising an Fc portion with particles. The present invention further relates to the use of a system comprising an Fc-binding peptide comprising a fluorescent label, a protein comprising an Fc portion which is to be bound by an Fc-binding peptide and a device configured to analyse alterations in the fluorescence spectrum of the labelled protein comprising an Fc portion upon a potential interaction of the labelled protein comprising an Fc portion with particles for measuring an interaction between a fluorescently labelled protein comprising an Fc portion and particles in a solution. The aforementioned methods make use of analysing alterations in the fluorescence spectrum of said labelled protein comprising an Fc portion upon a potential interaction with said particles.

In former methods for all-optical biomolecule characterization, samples with biomolecules in solutions were homogeneously heated to a certain temperature at a time followed by further heating to the next temperature point, which, however, was time-consuming. Only then fluorescence was measured. Duhr et. al. in European Phys. J. E 15,277, 2004 relates to "Thermophoresis of DNA determined by microfluidic fluorescence" and makes use of thermophoretic driving forces in miniaturized biotechnology devices. The temperatures were measured with high spatial resolution by the temperature sensitive fluorescence of a fluorescent dye. Typically, one measurement according to Duhr et al. (2004, loc. cit.) takes 300s or even more. Those older methods were improved by thermo-optical characterization methods like microscale thermophoresis (MST) which is based on the absorbance of infrared laser radiation by aqueous solutions and the subsequent conversion into heat (see, e.g., WO 2008/061706). Thereby it is possible to create broad spatial, i.e., two-dimensional orthree-dimensional (2D, 3D), temperature distributions comprising all desired temperatures, e.g. between 0°C and 100°C within an area of e.g. about 250 µm in diameter or length, established by local laser heating, whereby desired temperature gradients are created, in particular strong temperature gradients.

Methods known in the art for measuring inter- and/or intramolecular interactions of particles and/or modifications of particles rely on Solvent-sensitive fluorescent dyes, namely merocyanine dyes, as they are known to be suitable for imaging in vivo by covalent attachment to proteins. These dyes can be used to study conformational changes of proteins (Hahn et al. in J. Am. Chem. Soc. 2003, 125, 4132-4145). Polymethine dyes have been reported to be useful as spectral-fluorescent probes for studying noncovalent interactions of these dyes with biomacromolecules such as nucleic acids and proteins (Tatikolov in Journal of Photochemistry and Photobiology C: Photochemistry Reviews 2012, 13, 55-90). The photophysical properties of cyanine dyes are affected by the molecular environment within a biomolecule, thus making these dyes suitable as fluorescent probes in biophysical research (Levitus et al. in Quarterly Reviews of Biophysics 2010, 1-29). Those methods have been improved by methods for measuring inter- and/or intramolecular interactions of particles and/or modifications of particles with increased sensitivity and being independent of the source of heating and the geometry of the reaction vessel. Such improved methods are based on temperature-related intensity change (TRIC) and described, e.g., in WO 2018/234557. Such methods are also suitable for cases with a small absolute intensity variation at room temperature. However, an increase in temperature, even by only a few degrees Celsius is not always tolerated by the particles to be measured and the fluorescence intensity may equally change for the fluorescently labeled particle and for the fluorescently labeled particle complexed with a ligand. Also, inhomogeneous samples such as samples containing a certain fraction of aggregates, can lead to irreproducible fluorescence traces due to convection, making it impossible to obtain binding curves for systems with small signal amplitudes.

The fluorescence spectrum of a fluorescent label is sensitive to environmental changes such as changes in the chemical surrounding and temperature changes. Thus, the same fluorescent label can show alterations in its fluorescence spectrum in terms of intensity and/or spectral shifts and/or spectral shape. Since this effect is well-known, it is used to study interactions of intrinsically or extrinsically fluorescently labeled particles. Conformational changes of proteins and analyte concentrations are determined in the art based on methods involving a mechanism called Förster resonance energy transfer (FRET) (WO 2017/087912). The fundamental mechanism of FRET involves a donor fluorophore in an excited electronic state, which may transfer its excitation energy to a nearby acceptor fluorophore through dipole-dipole coupling due to interaction-mediated (e.g., ligand-mediated) changes in the distance and/or the angle between the two fluorophores. As such, FRET measurements require two or more fluorescent labels, i.e. at least one donor and at least one acceptor fluorophore. However, since two different fluorescent labels each of which may have different sensitivities to environmental changes are used, FRET measurements may be falsified by undesired changes in the local environment of the fluorophores, thus also leading to low resolution of spectrophotometric measurements. As a consequence, improved methods were developed for characterization of inter- and/or intramolecular interactions, and/or modifications (conformational changes) and/or (changes in) localization of fluorescently labeled particles, including, e.g., spectral shift technology (SPS) as described in, e.g., WO 2023/275274. This method provides an improved sensitivity in detecting alterations in the fluorescence spectrum of fluorescent labels within small sample volumes of fluorescently labeled particles.

Many methods known in the art which are based on measuring fluorescence emission rely on the Stoke shift which basically describes the wavelength shift of light between absorption and emission. Generally, the wavelength of light which is emitted from fluorescent particles is longer than the wavelength of the previously absorbed light. In other words, the emitted photon has less energy than the previously absorbed photon. The energy difference between the emitted photon and the absorbed photon is the Stokes shift which can be measured.

Methods known in the art and described above use the phenomenon of the Stokes shift when labelling a particle to be characterized in terms of its thermos-optical properties (e.g., microscale thermophoresis MST), when directly labeling a binding agent (e.g., an antibody) to measure interactions between the labelled binding agent and its ligand (e.g., temperature-related intensity change TRIC), or when labeling particles to analyze fluorescence spectrum alterations at different wavelengths upon excitation with a shorter wavelength (e.g., spectral shift technology SPS).

However, such methods where the fluorescent dye was directly coupled with the particle to be analyzed or to the binding agent, e.g. an antibody whose interaction with a particle, e.g. its ligand should be measured did not always result in satisfying measurements of spectrum alterations upon binding of a ligand.

These and further disadvantages need to be overcome. The present invention therefore addresses these needs and technical objectives and provides a solution as described herein, and as defined in the claims as well as exemplified in the appended examples and illustrated in the figures.

Accordingly, the present invention relates to a method for measuring an interaction between a fluorescently labelled protein comprising an Fc portion and one or more particles in a solution, comprising analysing alterations in the fluorescence spectrum of said labelled protein comprising an Fc portion upon a potential interaction with said particles, wherein said protein comprising an Fc portion is fluorescently labelled by a Fc-binding peptide capable of binding to said protein comprising an Fc portion within its Fc region, said Fc-binding peptide comprising a fluorescent label. Thus, a protein comprising an Fc portion is in the context of the present invention, so to say, indirectly labelled through binding of a labelled Fc-binding peptide to said protein comprising an Fc portion.

Previously known methods for measuring an interaction between a protein comprising an Fc portion and particles as described herein above comprised labelling the protein comprising an Fc portion directly with a dye, e.g., via NHS- or maleimide linkers. In contrast, as has been surprisingly found in context with the present invention, labelling a protein comprising an Fc portion (e.g., antibody) via an Fc-binding agent, said Fc-binding agent being itself directly fluorescently labelled, allows detection of alterations in the fluorescence spectrum with substantially higher sensitivity.

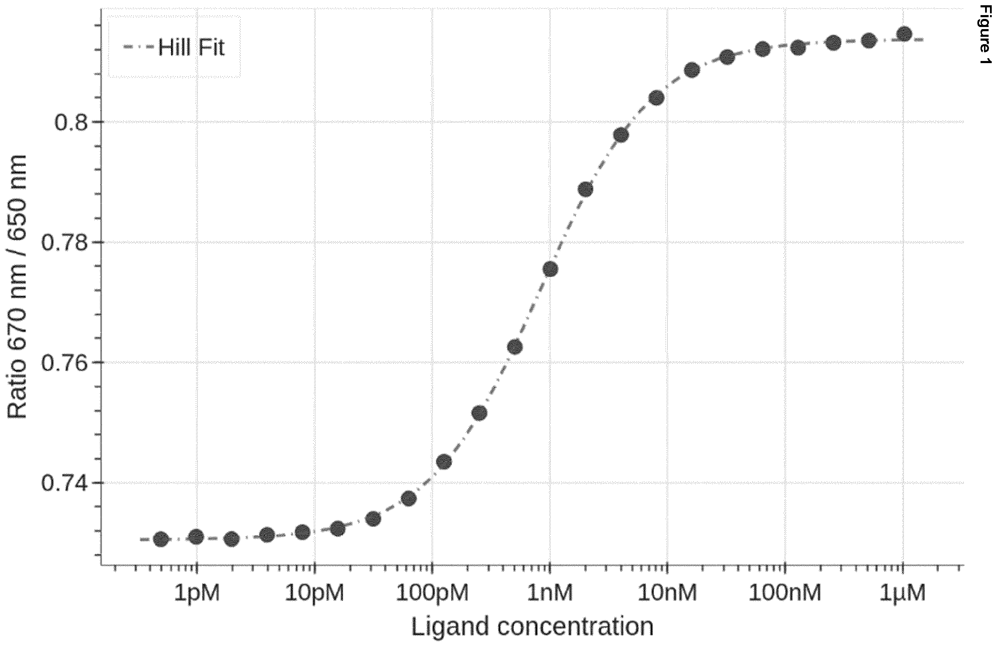

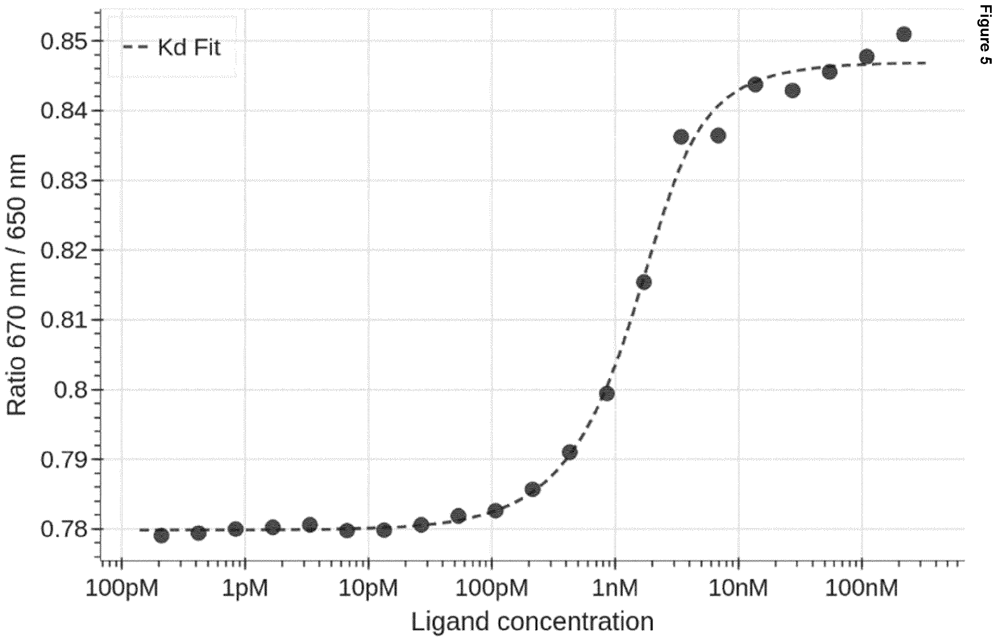

In other words, as has been found by the present invention, by using fluorescently labeled proteins, one can specifically label proteins comprising an Fc portion (e.g., antibody) with fluorescent molecules (e.g. Fc-binding peptide comprising a fluorescent label), and in a second subsequent step detect ligand binding to said label protein comprising an Fc portion (e.g., antibody) using, e.g., MST, TRIC and/or spectral shift signals. Accordingly, for example, by way of the present invention, the affinity of the fluorescent peptide to the label protein comprising an Fc portion (e.g., antibody) may be determined by dilution experiments and measured in MST, TRIC, SpS (cf., e.g., Figures 1 - 4). Also, e.g. the fluorescent molecule (e.g. Fc-binding peptide comprising a fluorescent label) may be incubated with appropriate amounts of a protein comprising an Fc portion (e.g., antibody) and ligand binding assays may be performed by dilution series of the latter (cf., e.g., Figures 5-8). This dilution series may then be measured using, e.g., MST, TRIC, and/or SpS and the resulting dose-response curve may be used to determine the affinity of the ligand to the label protein comprising an Fc portion (e.g., antibody). This makes this approach highly suitable inter alia for ligand screening purposes. This finding of the present invention was indeed surprising as the skilled person would have expected that a dye being more remote to the actual binding site between the protein comprising an Fc portion and the particle which the protein comprising an Fc portion binds to would not show a suitable spectral shift signal. Rather, the skilled person would have expected that it would be beneficial to receive a strong spectral shift signal if the dye was closer to the actual binding site between the protein comprising an Fc portion and the particle which the protein comprising an Fc portion binds to, i.e. when the dye was directly labelled to the protein comprising an Fc portion binding to the particle instead - as is the case in accordance with the present invention - of being labelled to an Fc-binding peptide (which in turns binds to the Fc-region of the protein comprising an Fc portion), thus being more remote to the actual binding site between the protein comprising an Fc portion and the particle which the protein comprising an Fc portion binds to.

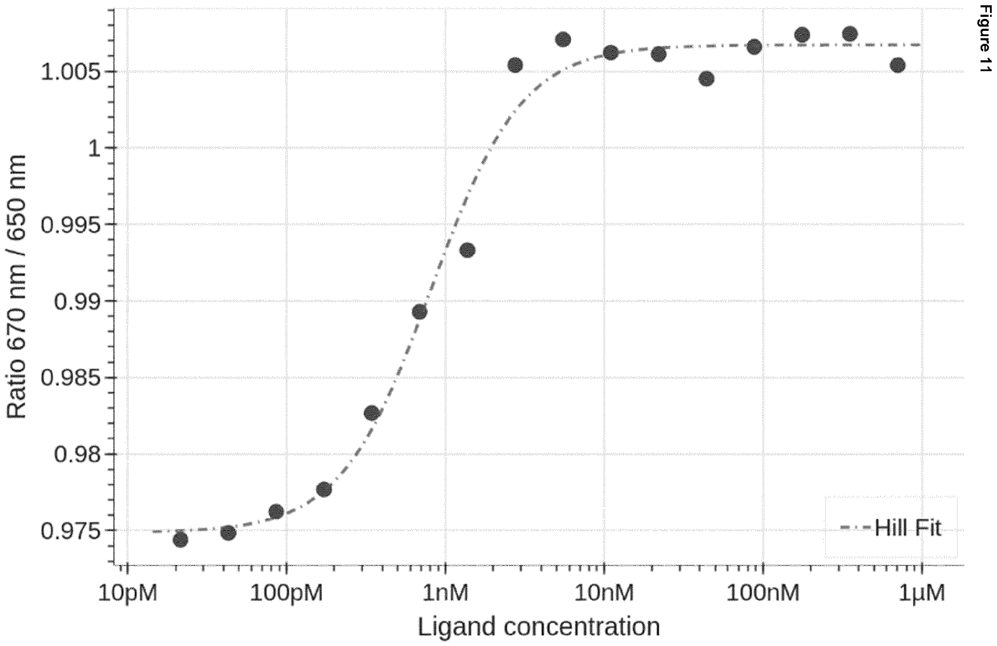

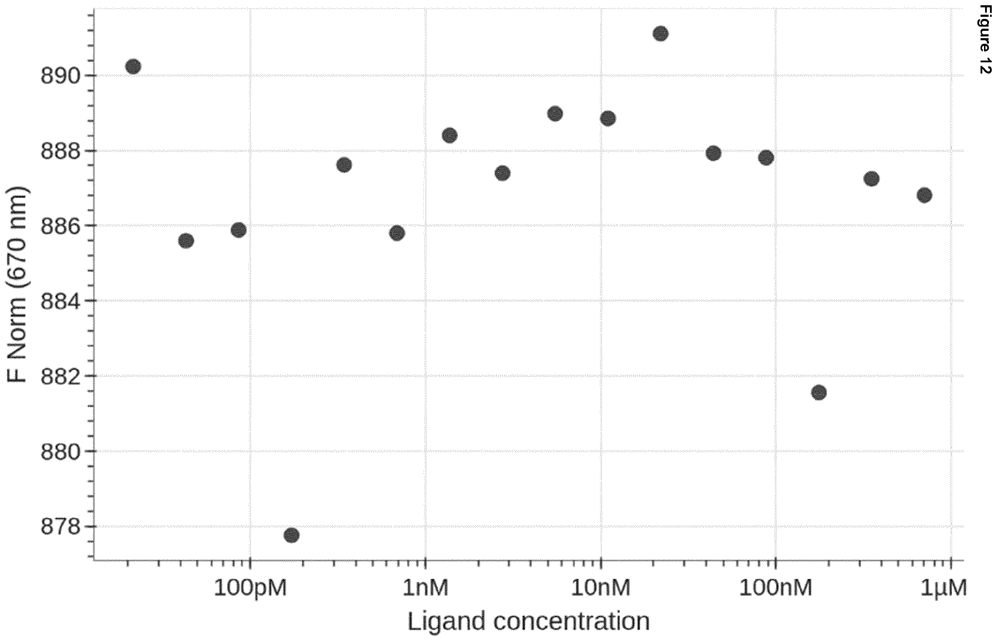

Overall, as also described herein and shown in the examples, using a fluorescently labeled protein according to the present invention to label the Fc part of proteins comprising an Fc portion (e.g., an antibody, for example human IgG) allows to follow ligand binding (e.g., Her2) to the protein comprising an Fc portion (e.g., antibody) with high signal-to-noise values in both, ratiometric fluorescence changes (spectral shift, SpS) and temperature related intensity change (TRIC) (cf. Fig. 5-8). In contrast, traditionally labeled antibodies, e.g. Cy5-NHS although showing a dose-response signal (cf. Fig. 11), show much lower sensitivity and overall signal. Additionally, as shown in the examples herein, no dose-response could be observed for the TRIC signal (cf. Fig. 12). Moreover, binding assays performed via the herein described method can be performed using a mix-and-measure approach, as no purification of the antibody is necessary, e.g., to remove unbound fluorescent dye. Taken together, these results show the major advantages of Fc-protein labeling according to the present invention.

The term "peptide" is equally used herein with the term "protein". Proteins (including fragments thereof, preferably biologically active fragments, and peptides, usually having less than 30 amino acids) comprise one or more amino acids coupled to each other via a covalent peptide bond (resulting in a chain of amino acids). The term "peptide" as used herein describes a group of molecules which typically comprise more than 2 amino acids and usually not more than 30 amino acids. Polypeptides may further form multimers such as dimers, trimers and higher oligomers, *i.e.* consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hetero-multimer is an antibody molecule, which, in its naturally occurring form, consists of two identical light polypeptide chains and two identical heavy polypeptide chains. The terms "peptide" and "protein" also refer to naturally modified polypeptides/proteins wherein the modification is effected e.g. by post-translational modifications like glycosylation, acetylation, phosphorylation and the like. Such modifications are well known in the art. A preferred example of a peptide as described above is a Fc-binding peptide.

The term "protein comprising an Fc portion" as used herein comprises all proteins as defined herein which comprise at least a portion of the Fc (fragment crystallizable) part of an antibody, preferably the full Fc part of an antibody. The term "Fc part" - or, as used interchangeably herein, the "Fc region" - is generally known in the art and encompasses, e.g., the part of antibodies that are located distal of the binding regions of antibodies (e.g., Fab) and which interact with Fc receptors on cell surfaces. Typically, antibodies (e.g., IgG, IgA or IgD) comprise two, normally identical protein fragments as part of the constant domains of the heavy chains, together constituting the Fc part or Fc region. A "protein comprising an Fc portion" comprises at least a portion of one of those protein fragments that together constitute the Fc part, but may also comprise one full of those protein fragments, optionally plus at least a portion of the other of those protein fragments. In one embodiment of the present invention, said protein comprising an Fc portion as used herein comprises both of those protein fragments that together constitute the Fc part.

In a specific embodiment of the present invention, the protein comprising an Fc portion as used herein is an antibody. Without being bound by theory, it seems that, apart from labelling an Fc containing protein, e.g. an antibody pursuant to the teaching of the present invention, in case of an antibody it appears advantageous to label an antibody pursuant to the teaching of the present invention distant, i.e., within its Fc portion, from its VH and VL regions which are, so to say, the binding region of the antibody.

An "antibody" when used herein is a protein comprising one or more polypeptides (comprising one or more binding domains, preferably antigen binding domains) substantially or partially encoded by immunoglobulin genes or fragments of immunoglobulin genes and comprises at least one Fc region or parts thereof as long as such parts are bound by said Fc-binding peptide. The skilled person is readily in a position to determine whether or not a part of a Fc region may be bound by a Fc-binding peptide. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, lgA1, and IgA2. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 Daltons (Da).

Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen.

When used herein the term "antibody" does not only refer to an immunoglobulin (or intact antibody), but also to a fragment thereof, and encompasses any polypeptide comprising an antigen-binding fragment or an antigen-binding domain which still contains at least one Fc region, e.g., scFv-Fc, IgNAR or hclgG fragments. Typically, such fragments would comprise an antigen-binding domain and have the same properties as the antibodies described herein.

The term "antibody" also includes but is not limited to polyclonal, monoclonal, monospecific, polyspecific such as bispecific, non-specific, humanized, human, single-chain, chimeric, synthetic, recombinant, hybrid, mutated, grafted, and in vitro generated antibodies, with a polyclonal antibody being preferred. Said term also includes domain antibodies (dAbs) and nanobodies. A "bispecific" or "bifunctional antibody" is an artificial hybrid antibody having two different heavy/light chain pairs and two different binding sites. Bispecific antibodies can be produced by a variety of methods including fusion of hybridomas or linking of binding fragments. See, e.g., Songsivilai & Lachmann, Clin Exp Immunol (1990), 79: 315-321; Kostelny et al., J Immunol (1992), 148: 1547-1553.

The term "antibody" as used herein comprises antibodies as described herein, from any origin, e.g., human, mouse, rat, rabbit, goat, camel, llama, and others, and also includes in vitro-derived antibodies and modified antibodies such as humanized antibodies. "Humanized" forms of non-human (e.g., murine) antibodies are chimeric immunoglobulins, immunoglobulin chains or fragments thereof of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat, rabbit, goat, camel, llama, and others, having the desired specificity, affinity, and capacity. Furthermore, "humanized antibodies" as used herein may also comprise residues which are found neither in the recipient antibody nor the donor antibody. These modifications are made to further refine and optimize antibody performance. The humanized antibody as used herein also comprise at least a portion of an immunoglobulin constant region (Fc), typically that of a human immunoglobulin. For further details, see Jones et al., Nature (1986), 321: 522-525; Reichmann et al., Nature (1988), 332: 323-329; and Presta, Curr. Op. Struct Biol (1992), 2: 593-596. As used herein, "in vitro derived antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

An Fc-binding peptide as applied in the context of the present invention is known in the art and further described herein. In the context of the present invention, not only one, but also two or more Fc-binding peptides may be applied. For example, if two or more Fc-binding peptides are applied, Fc-binding peptides should be able to bind to two of the same binding interfaces on the Fc stalk. For example, if two or more Fc-binding peptides are applied, both may be fluorescently labelled. Preferably, two different fluorophores, blackhole quencher or FRET pairs may be used, e.g. to measure e.g. bispecific antibodies.

Preferably, when two or more Fc-binding peptides are applied, a first Fc-binding peptide binds to a first portion within an Fc region of the antibody and a second Fc-binding peptide binds to a second portion within an Fc region of said antibody, wherein the first and second portion are different.

As used herein and unless specified otherwise, the term "antibody" also comprises chemoimmuno-conjugates, particularly antibody-drug-conjugates (ADC) as known to the skilled person, where, e.g., chemotherapeutic agents are linked to antibodies, e.g., cytostatic drugs or cytotoxic agents.

The present invention thus also relates to a method for measuring an interaction between a fluorescently labelled protein comprising an Fc portion (e.g., antibody) and one or more particles in a solution, comprising analysing alterations in the fluorescence spectrum of said labelled protein comprising an Fc portion (e.g., antibody) upon a potential interaction with said particles, wherein said protein comprising an Fc portion (e.g., antibody) is fluorescently labelled by a Fc-binding peptide capable of binding to said protein comprising an Fc portion (e.g., antibody) within its Fc region, said Fc-binding peptide comprising a fluorescent label, wherein at least one labelled Fc-binding peptide binds to a first of two same binding sites within the Fc region of said protein comprising an Fc portion (e.g., antibody), and at least one labelled Fc-binding peptide binds to a second of two same binding sites within the Fc region of said protein comprising an Fc portion (e.g., antibody), wherein the first and the second binding sites within the Fc region of said protein comprising an Fc portion (e.g., antibody) are different, and preferably wherein the labelled Fc-binding peptide binding to the first binding sites within an Fc region comprises a different label than the Fc-binding peptide binding to the second binding sites within an Fc region. This method is particularly considered useful to measure interactions of bispecific antibodies with one or more particles. Accordingly, in this context, said protein comprising an Fc portion may be an antibody and said antibody may be bispecific.

According to the present invention, the term "particles" includes molecules, in particular organic molecules, biomolecules, nanoparticles, microparticles and vesicles. The term "particles" also includes biological cells (e.g., bacterial or eukaryotic cells) or subcellular fragments, biological tissues, viral particles, virus-like particles or viruses and cellular organelles, lipid nanoparticles (LNPs) and the like. Nanoparticles also include nanodiscs. A nanodisc is a synthetic model membrane system composed of a lipid bilayer of phospholipids with the hydrophobic edge screened by two amphipathic proteins. Biomolecules are preferably selected from the group consisting of amino acids, proteins, peptides, mono- and disaccharides, polysaccharides, lipids, glycolipids, fatty acids, sterols, vitamins, neurotransmitter, enzymes, nucleotides, metabolites, nucleic acids, and combinations or complexes thereof. More preferably, the biomolecules are selected from the group consisting of proteins, peptides, enzymes, nucleic acids, and combinations or complexes thereof. For example, the particles (in the labeled particles) are biomolecules, most preferably proteins or nucleic acids. The proteins may be selected from the group consisting of enzymes ( e.g., carbonic anhydrase, beta lactamase TEMI, or kinases such as MEKI and p38), transporter proteins (e.g., MBP), inhibitory proteins (e.g., beta lactamase inhibitory protein BLIP, Anakinra), structural proteins, signaling proteins, ligand-binding proteins, chaperones ( e.g., heat shock protein HSP90), antibodies (e.g., Trastuzumab), membrane proteins, and receptors (e.g., interleukin 1 receptor). Nucleic acids include DNA, RNA ( e.g. mRNA, tRNA, rRNA and the like ), LNA and PNA. Also, modified ( e.g. chemically modified) nucleic acids can be analyzed in the context of the present invention. Locked nucleic acid (LNA), often referred to as inaccessible RNA, is a modified RNA nucleotide. The ribose moiety of an LNA nucleotide is modified with an extra bridge connecting the 2' oxygen and 4' carbon. Peptide nucleic acid (PNA) is an artificially synthesized polymer similar to DNA or RNA. DNA and RNA have a deoxyribose and ribose sugar backbone, respectively, whereas PNA's backbone is composed of a peptide such as repeating N-(2-aminoethyl)-glycine units linked by peptide bonds. The various purine and pyrimidine bases are linked to the backbone by a methylene bridge (-CH2-) and a carbonyl group (-(C=O)-).

In some aspects of the present invention, the particles are selected from the group consisting of organic molecules, biomolecules, nanoparticles, microparticles, vesicles, biological cells or sub-cellular fragments, biological tissues, viral particles, viruses, cellular organelles, lipid nanoparticles (LNPs ), and virus like particles. In some aspects of the present invention, the biomolecules are selected from the group consisting of amino acids, proteins, peptides, mono- and disaccharides, polysaccharides, lipids, glycolipids, fatty acids, sterols, vitamins, neurotransmitter, enzymes, nucleotides, metabolites, nucleic acids, and combinations thereof.

In the context of the present invention, a nanoparticle may be a particle having an average size of less than 100 nm. The term "average size" describes the mean effective diameter as measured by dynamic light scattering using, for example, Brookhaven Instruments' 90Plus or Malvem Zetasizer Z90 particle sizing instrument. For example, the nanoparticle size is in the range of 1 nm to 100 nm, preferably 1 to 70 nm. The nanoparticles can be organic or inorganic particles. The nanoparticles can also be present as composite particles, such as an inorganic core having organic molecules attached to its surface. A microparticle is a microscopic particle which has a longest dimension of less than 1 mm but normally more than 100 nm. Sizing methods employing transmission electron microscopy (TEM), scanning electron microscopy (SEM), and quasi-elastic light scattering (QELS) may be used to characterize the microparticle. The microparticles can also be present in the form of microbeads. The microparticles can be, e.g., coated or uncoated silica-/glass-/biodegradable particles, polystyrene-/coated-/flow cytometry-/PMMA-/melamine-/NIST particles, agarose particles, magnetic particles, coated or uncoated gold particles or silver particles or other metal particles, transition metal particles, biological materials, semiconductors, organic and inorganic particles, fluorescent polystyrene microspheres, non-fluorescent polystyrene microspheres, composite materials, liposomes, cells and the like.

Commercially available microparticles are available in a wide variety of materials, including ceramics, glass, polymers, and metals. Microparticles encountered in daily life include pollen, sand, dust, flour, and powdered sugar. In biological systems, microparticles are small membrane bound vesicles circulating in the blood derived from cells that are in contact with the bloodstream, such as platelets and endothelial cells.

Microbeads may inter alia be manufactured solid plastic particles of less than 5 mm in their largest dimension. Microbeads may also be uniform polymer particles, typically 0.5 to 500 µm in diameter. The term "modified particle" or "modified bead" relates, in particular, to beads or particles which comprise or are linked to molecules, preferably biomolecules. This also comprises the coating of such beads or particles with these (bio )molecules. Particles or beads according to this invention may be modified in such a way that, for example, biomolecules, e.g., DNA, RNA or proteins, may be able to bind (in some embodiments specifically and/or covalently) to the particles or beads. Therefore, within the scope of this invention is the analysis of characteristics of beads and/or particles and in particular of molecules attached to or linked to such beads or particles. In particular, such molecules are biomolecules. Accordingly, the term "modified (micro)beads/(nano- or micro)particles", in particular, relates to beads or particles which comprise additional molecules to be analyzed or characterized. Modified or non-modified microparticles/(nano- or micro)particles may be able to interact with other particles/molecules such as biomolecules (e.g., DNA, RNA or proteins) in solution.

The method described and provided in context with the present invention may generally be applied in connection with known methods suitable to measure alterations in the fluorescence spectrum, e.g., those being based on measuring fluorescence emission relying on the Stoke shift. For example, in one embodiment of the present invention, the alterations in the fluorescence spectrum of said labelled protein comprising an Fc portion (e.g., antibody) may be analysed by microscale thermophoresis, temperature-related intensity change or spectral shift technology as described in WO 2008/061706, WO 2018/234557, and WO 2023/275274, respectively.

Accordingly, in one embodiment of the present invention, the alterations in the fluorescence spectrum which are analysed upon a potential interaction between said protein comprising an Fc portion (e.g., antibody) and said particle(s) may be alterations in the fluorescence emission spectrum. Such alterations may include, e.g., spectral shifts like bathochromic (red) shifts or hypsochromic (blue) shifts. In the context of the present invention, the magnitude of the spectral shifts is preferably at least 50 pm, more preferably at least 100 pm, even more preferably at least 500pm.

According to the present invention, the fluorescence intensity of the fluorescently labeled Fc-binding peptides (or particles) preferably changes due to mechanisms selected from the group consisting of conformational changes of the fluorescently labeled Fc-binding peptides (or particles), re-localization of the fluorescently labeled Fc-binding peptides (or particles), interactions between the fluorescently labeled Fc-binding peptides (or particles) and one or more ligands or combinations thereof and the like.

In one embodiment of the present invention, the labelled protein comprising an Fc portion (e.g., antibody) may be dissolved or dispersed in the solution or may be immobilized on a solid support. In another embodiment of the present invention, the particles as described herein may be dissolved or dispersed in the solution or may be immobilized on a solid support. In a specific embodiment of the present invention, the labelled protein comprising an Fc portion (e.g., antibody) and the particles may be dissolved or dispersed in the solution. In another specific embodiment of the present invention, the labelled protein comprising an Fc portion (e.g., antibody) may be dissolved or dispersed in the solution, and the particles may be immobilized on a solid support. In another specific embodiment of the present invention, the labelled protein comprising an Fc portion (e.g., antibody) may be immobilized on a solid support, and the particles may be dissolved or dispersed in the solution. Examples for solid support suitable to be employed in accordance with the present invention include, e.g., the surface of micro- or nanoparticles as described herein (e.g., lipid based, synthetic, protein based, viral - mixed lipid and protein), or glass/polystyrene beads. In one embodiment of the present invention, surfaces (preferably solid surfaces) are coated with proteins comprising an Fc portion (e.g., antibodies) or particles as described herein.

In one embodiment of the present invention, said solution may further comprise a compound which may suppress, inhibit or enhance an interaction between said labelled protein comprising an Fc portion (e.g., antibody) and said particles.

In one embodiment of the present invention, an alteration in the fluorescence spectrum of said labelled protein comprising an Fc portion (e.g., antibody) may be indicative of an interaction between said labelled protein comprising an Fc portion (e.g., antibody) and said particles. Alterations in the fluorescence spectrum of a fluorescent label are usually detected with fluorescence spectrophotometers, which are designed to record/measure over a large spectral range and thus, fail to resolve small changes in the fluorescence intensity. For example, in accordance with the present invention, alterations in the detected fluorescence intensity of the fluorescently labeled particles result from spectral shifts or broadening of the spectrum or narrowing of the spectrum, or combinations thereof, preferably from spectral shifts.

In one embodiment of the present invention, as a control the fluorescence spectrum of said labelled protein comprising an Fc portion (e.g., antibody) may be analysed in the absence of said particles.

In one embodiment of the present invention, said particles may be selected from the group consisting of organic molecules, biomolecules, nanoparticles, microparticles, vesicles, biological cells or sub-cellular fragments, biological tissues, viral particles, viruses, cellular organelles, lipid nanoparticles (LNPs), and virus like particles. For example, in accordance with the present invention, said biomolecules may be selected from the group consisting of amino acids, proteins, peptides, mono- and disaccharides, polysaccharides, lipids, glycolipids, fatty acids, sterols, vitamins, neurotransmitter, enzymes, nucleotides, metabolites, nucleic acids, and combinations thereof.

The term "interaction(s)" as used herein comprises inter alia interaction(s) between biomolecules (e.g., protein, DNA, RNA, hyaluronic acids etc.) but also between (modified) (nano)particles/(micro)beads and biomolecules. Also within the scope of the present invention is, e.g., the measurement of protein-protein interactions, like complex formations of proteinaceous structures or of proteins or of fragments thereof. Such measurements comprise, but are not limited to, the measurement of antibody-antigen binding reactions (also in form of single chain antibodies, antibody fragments, chromobodies and the like). Yet, the embodiments of the present invention are also related to the detection and or measurement of dissociation events, like, e.g., the dissociation of protein complexes. Therefore, the invention is also useful in the measurement, determination and/or verification of dissociation events, like in the measurement of the dissociation of proteinaceous complexes, e.g., antibody-antigen complexes and the like.

Thus, in one embodiment of the present invention, said interaction may be binding between said protein comprising an Fc portion (e.g., antibody) and said particles.

In one embodiment of the present invention, said particles comprise a protein comprising an epitope for said labelled protein comprising an Fc portion (e.g., antibody). In this context, in one embodiment of the present invention, it is envisaged that said labelled protein comprising an Fc portion (e.g., antibody) interacts with said epitope.

In one embodiment of the present invention, said particles may comprise the same protein comprising an Fc portion (e.g., antibody) as the fluorescently labelled protein comprising an Fc portion (e.g., antibody), with the exception that it does not comprise the same fluorescent label as said fluorescently labelled protein comprising an Fc portion (e.g., antibody).

In the methods of the present invention, also labeled particles may be employed which are labeled with at least one fluorescent label, preferably exactly one fluorescent label, wherein the label of the particle is not the same as the label comprised by the Fc-binding peptide capable of binding within the Fc region of said protein comprising an Fc portion (e.g., antibody) whose interaction with said particle(s) is to be measured. In the context of this invention, particles labeled with more than one fluorescent label are labeled with only one type of fluorescent label (i.e. only a single type of label per particle). In the context of this invention, "labeled peptides" or "labeled particles" refer to fluorescently labeled peptides or particles which can be detected by fluorescence means, e.g., molecules/particles comprising an intrinsic fluorophore, or particles/molecules tagged with fusion proteins or particles/molecules with extrinsic fluorophores attached. In particular, such labeled peptides or particles may be which are attached, e.g., covalently bonded to a label ( e.g. via NHS labeling, maleimide labeling and the like ), reversibly bonded to a label over a high affinity protein tag, e.g. HIS-tag, AVI-tag, SPOT-tag, SNAP-tag and the like or bioconjugated via Copper(I)-catalyzed azide-alkyne cycloaddition (CuAAC), Strain promoted azide-alkyne cycloaddition (SPAAC) and the like (also known as click-chemistry) or similar.

In one embodiment of the present invention, said Fc-binding peptide may be non-covalently bound to said protein comprising an Fc portion (e.g., antibody).

In one embodiment of the present invention, said Fc-binding peptide may remain bound to said protein comprising an Fc portion (e.g., antibody) up to a temperature of at least about 60, 65, 70, 75, 80, 85, 90, 95 or 100°C. For example, said Fc-binding peptide may remain bound to said protein comprising an Fc portion (e.g., antibody) up to a temperature of at least about 60 °C.

In context with the present invention, and unless specified herein otherwise, affinity of a binding protein (protein comprising an Fc portion, e.g., antibody) is preferably measured as dissociation constant (Kd). In one embodiment of the present invention, said Fc-binding peptide may have an affinity to said protein comprising an Fc portion (e.g., antibody) of about 1×10⁻⁷ M or higher. For example, in accordance with the present invention, particularly where said protein comprising an Fc portion is an IgG antibody, said Fc-binding peptide may have an affinity to said protein comprising an Fc portion (e.g., IgG antibody) of at least about 10⁻⁷ M, at least about 25 nM, at least about 20 nM, at least about 16 nM, at least about 2.5 nM, at least about 1.8 nM, or at least about 0.5 nM. In another example, in accordance with the present invention, particularly where said protein comprising an Fc portion is an IgA antibody, said Fc-binding peptide may have an affinity to said protein comprising an Fc portion (e.g., IgA antibody) of at least about 10⁻⁷ M, at least about 72 nM, at least about 33 nM, or at least about 16 nM.

In one embodiment of the present invention, said Fc-binding peptide may comprise about 4 to 30 amino acids, for example about 5 to 20, about 5 to 18, about 5 to 16 or about 7 to 15 amino acids.

In one embodiment of the present invention, said Fc-binding peptide may comprise at least one linker. In this context, in one embodiment of the present invention, said linker comprised by said Fc-binding peptide may be activatable. In accordance with the present invention, said linker comprised by said Fc-binding peptide may comprise at least one moiety which allows covalent binding with an amino acid or peptide within the Fc region of said protein comprising an Fc portion (e.g., antibody) upon binding of said Fc-binding peptide to said protein comprising an Fc portion (e.g., antibody). Said Fc-binding peptide may be linked to said antibody by methods known in the art, e.g., covalent binding to said protein comprising an Fc portion (e.g., antibody) or via linkers (e.g., photoactivated linkers, unspecific crosslinking, or the like). Methods for site-specific covalent binding of an amino acid or a peptide to the Fc region are known in the art (see, e.g., Kishimoto et al., Bioconjugate Chem (2019), 30(3): 698-702). Suitable linkers are known in the art and described, e.g., in Harroun et al., Nature (2022), 19(1): 71-80.

In one embodiment of the present invention, the Fc-binding peptide is Fclll or a variant or derivative thereof (Fclll see also. DeLano et al., Science (2000), 287: 1279-1283).

In one embodiment of the present invention, said protein comprising an Fc portion (e.g., antibody) may be an IgG antibody. In accordance with the present invention, particularly in the embodiment where said protein comprising an Fc portion (e.g., antibody) is an IgG antibody, said Fc-binding peptide may be or have a sequence of any of
(a) DCAWHLGELVWCT (SEQ ID NO: 1; Fc-III / 16-25 nM); cf. DOI: 10.1126/science.287.5456.1279),
(b) L-Pro DCAWHLGELVWCT D-Pro (SEQ ID NO: 2; FcBP2 / 1.8 nM cf. DOI: 10.1021/ja057513w),
(c) CDCAWHLGELVWCTC (SEQ ID NO: 3; Fc-III-4C / 2.5 nM cf. DOI: 10.1021/acs.bioconjchem.6b00170),
(d) RRGW (SEQ ID NO: 4; 0.5 nM; cf. DOI: 10.1021/ac4029467),
(e) KHRFNKD (SEQ ID NO: 5; 20 nM; cf. DOI: 10.1007/s13206-016-0202-z), or
(f) or any one of Fc-binding peptides as shown in doi:10.3390/ma9120994.

In one embodiment of the present invention, said protein comprising an Fc portion (e.g., antibody) may be an IgA antibody. In accordance with the present invention, particularly in the embodiment where said protein comprising an Fc portion (e.g., antibody) is an IgA antibody, said Fc-binding peptide may be or have a sequence of any of
(a) HMVCLAYRGRPVCFAL (SEQ ID NO: 6; 33 nM; cf. doi: 10.1074/jbc.M112.389742),
(b) HMVCLSYRGRPVCFSL (SEQ ID NO: 7; 16 nM; cf. doi: 10.1074/jbc.M112.389742), or
(c) HQVCLSYRGRPVCFST (SEQ ID NO: 8; 72 nM; cf. doi: 10.1074/jbc.M112.389742).

An Fc-binding peptide as described herein comprises a fluorescent label. In one embodiment of the present invention, said fluorescent label may be a fluorescent dye. In accordance with the present invention, examples for such fluorescent dyes may comprise Cy5, Cy3, Atto647, Atto647N, Alexa647, Dy647, and variants thereof.

The label comprised by said Fc-binding peptide capable to bind within the Fc region of said protein comprising an Fc portion (e.g., antibody) may be attached to said Fc-binding peptide by any suitable method known in the art, e.g., covalently or non-covalently, preferably covalently, at the N- or C-terminus (preferably N-terminus). Also, in accordance with the present invention, the label may be attached to said Fc-binding peptide via a linker, e.g., NHS-maleimide esters or other linkers known in the art and as described, e.g., in Harroun et al., Nature (2022), 19(1): 71-80. The linker used in the context of the present invention is not limited by length (as long as the linker is long enough so that the fluorophore can reach to the target) and/or type of linker (as long as the type of linker has the property that its length can be adjusted ,e.g., DNA, peptide, amino acid, aromatic rings including aryl groups, PEG linkers, and the like) and any suitable linker known to the skilled person may be employed to indirectly attach a fluorescent label to the Fc-binding peptide (or particle) capable to bind within the Fc region of said protein comprising an Fc portion (e.g., antibody).

In the context of the present invention, the terms "label" and "dye" are used interchangeably and preferably refer to a fluorophore/fluorochrome, i.e., a fluorescent chemical compound, which is reemitting light upon excitation. Labels useful in the present invention comprise labels which are sensitive to environmental changes, i.e., the fluorescence spectrum of the dye alters upon environmental changes such as changes in the chemical microenvironment (ligand binding, conformational changes) and/or macroenvironment (e.g., location in LNPs versus location in cells) and/or temperature changes (e.g., heating or cooling).

Fluorescent labels for use according to the present invention can be selected from the group consisting of intrinsic fluorescent labels, fusion proteins, extrinsic fluorescent labels and the like. Intrinsic fluorescent labels include tryptophan residues, tyrosine residues, phenylalanine residues. Fusion proteins can be selected from the group consisting of blue-emitting fluorescent proteins, cyan-emitting fluorescent proteins, green-emitting fluorescent proteins, yellow emitting fluorescent proteins and red-emitting fluorescent proteins and the like. Reference is made to FPbase, a well-known data base in the art which provides a comprehensive list of presently known fluorescent proteins (https://www.fpbase.org/table/; Lambert, TJ (2019) FPbase: a community-editable fluorescent protein database. Nature Methods. 16, 277-278. doi:10.1038/s41592-019-0352-8).

In one embodiment of the present invention, the fluorescent labels may be extrinsic fluorescent labels. Extrinsic fluorescent labels can include but are not limited to commercially available labels, such as Cyanine dyes including Cy5, Cy3, Atto647, Atto647N, Alexa647 Dy647, and the like. Examples for extrinsic fluorescent labels are environment sensitive dyes described for example in WO 2018/234557, which is incorporated herein by reference. Environment sensitive dyes are known in the art and are described for example in Klymchenko, A. S. (2017) (Solvatochromic and fluorogenic dyes as environment-sensitive probes: design and biological applications. Accounts of chemical research, 50(2), 366-375.). In this context, WO 2018/234557 relates to fluorescent labels which are highly sensitive to environmental changes, e.g., changes in the chemical composition, temperature changes and the like. According to one embodiment of this invention, these dyes may be selected from the group consisting of the NanoTemper RED, GREEN, and BLUE dyes (commercially available e.g., as Protein Labeling Kits from NanoTemper Technologies GmbH, Munich, Germany).

By labeling with an extrinsic fluorescent label, it can be controlled that only one dye is attached to a target molecule and only that dye needs to be affected by ligand binding. The dye fluorescence range can be chosen so that it does not interfere with auto-fluorescence of a ligand. Lastly, extrinsic dyes are much brighter, and measurements can occur at much lower concentration of the target molecule, thus reducing sample consumption and allowing the measurement of even picomolar affinities.

Alterations in the fluorescence spectrum according to the present invention include changes in the fluorescence intensity of a fluorescent label, but also include spectral shifts and/or broadening or narrowing of their spectrum. According to the present invention, particularly where spectral shift technology is applied, it is preferred to detect the fluorescence at different wavelengths or wavelengths ranges, which could be achieved, e.g., by using bandpass filters. The detected intensities at these different wavelengths/wavelength ranges and the respective ratio allow a detection of a spectral shift of the entire emission spectrum, a broadening and/or narrowing of the spectrum. In this context, the spectral shifts preferably include bathochromic (i.e. red) shifts and/or hypsochromic (i.e. blue) shifts.

According to the present invention, the fluorescence intensity of the fluorescently labeled peptide (and/or particle) preferably changes due to mechanisms selected from the group consisting of conformational changes of the fluorescently labeled peptide (and/or particle), re-localization of the fluorescently labeled peptide (and/or particle), interactions between the fluorescently labeled peptide (and/or particle) and one or more ligands or combinations thereof and the like.

In one embodiment of the present invention, said alterations in the fluorescence spectrum of said labelled protein comprising an Fc portion (e.g., antibody) may be analysed by methods suitable to measure alterations in the fluorescence spectrum, e.g., those being based on measuring fluorescence emission relying on the Stoke shift. In one embodiment of the present invention, said alterations in the fluorescence spectrum of said labelled protein comprising an Fc portion (e.g., antibody) may be analysed by microscale thermophoresis, temperature-related intensity change or spectral shift technology. As an example, such alterations may be used to analyse unfolding of, e.g. an antibody. A further example for using such alterations is the analysis of unfolding of, e.g. an antibody in the presence or absence of the antigen bound by an antibody.

Further examples for dyes suitable to be employed in accordance with the present invention, particularly where temperature-related intensity change is used, are known in the art and described, e.g., in WO 2018/234557.

In accordance with the present invention, fluorescence can be measured as known by the skilled person. According to the present invention, preferable means for exciting, preferably fluorescently exciting the labeled Fc-binding peptides (or particles) may be any suitable device selected from the group consisting of laser, fibre laser, diode-laser, light emitting diodes (LEDs), Halogen, LED-Array, HBO (HBO lamps are, e.g., short arc lamps in which the discharge arc fires in an atmosphere of mercury vapor under high pressure ), HXP (HXP lamps are, e.g., short arc lamps in which the discharge arc bums in an atmosphere of mercury vapor at very high pressure e.g., in contrast to HBO lamps they are operated at a substantially higher pressure and they employ halogen cycle. HXP lamps generate UV and visible light, including significant portion of red light) and the like. Preferably, in the context of the present invention the excitation light source enables a highly focused excitation. In the context of the present invention, the excitation light source is preferably a laser, even more preferably an LED.

It is understood by the person skilled in the art that the term "fluorescence" as employed herein is not limited to "fluorescence" per se but that the herein disclosed means, methods and devices may also be used and employed by usage of other means, in particular luminescence, such as phosphorescence. The person skilled in the art is aware that in the context of this invention the "excitation" wavelength and the "emission" wavelengths have to be separated.

Although the analysis of fluorescently labeled particles according to the present invention does not necessarily rely on temperature induced fluorescence intensity changes, the fluorescence intensity measurement may be performed at a constant predetermined temperature or during a defined temperature perturbation. Accordingly, in accordance with the present invention, it is possible to apply a method for the characterization of fluorescently labeled peptides in solution by analyzing alterations in the fluorescence spectrum of the fluorescently labeled peptides in combination with a defined temperature perturbation. An example in this context is microscale thermophoresis as known in the art and described in, e.g., WO 2008/061706.

Accordingly, in one embodiment of the present invention, where said alterations in the fluorescence spectrum of said labelled protein comprising an Fc portion (e.g., antibody) are analysed by microscale thermophoresis, the microscale thermophoresis may comprise the following steps:
(a) providing a sample with said fluorescently labelled protein comprising an Fc portion (e.g., antibody) and particles in a solution;
(b) exciting said fluorescently labelled protein comprising an Fc portion (e.g., antibody) and firstly detecting fluorescence of said excited protein comprising an Fc portion (e.g., antibody);
(c) irradiating a laser light beam into the solution to obtain a spatial temperature distribution in the solution around the irradiated laser light beam;
(d) detecting secondly a fluorescence of the labelled protein comprising an Fc portion (e.g., antibody) in the solution at a predetermined time after irradiation of the laser into the solution has been started, and
(e) determining a potential interaction between said labelled protein comprising an Fc portion (e.g., antibody) and said particles based on said first and second detection.

In this context, where said alterations in the fluorescence spectrum of said labelled protein comprising an Fc portion (e.g., antibody) is analysed by microscale thermophoresis, the irradiation of the laser and the detection of the fluorescence may be conducted from the same side with respect to the sample. The heating or cooling can be carried out using a tempering element (i.e. a heating and/or cooling source) selected from the group consisting of heating and/or cooling fluids or gases, heating elements (for example a heating resistor, or other elements based on joule heating like Metal heating elements, Ceramic heating elements, Polymer PTC heating elements, Composite heating elements, semiconductor heating elements), or a thermoelectric element, for example a Peltier element, or electromagnetic radiation (like an LED, e.g., an IR-LED, or a laser, e.g., an IR laser, or a microwave). The use of an IR laser enables fast sample heating. A Peltier element is preferably used because it can be used to heat the sample and/or to cool the sample (e.g., to cool the sample below the environmental temperature). In particular, it is possible to switch from heating to cooling by reversing the direction of the current through the Peltier element. A Peltier element is one of the few elements that can heat but also actively cool under room temperature. A laser, preferably a laser whose electromagnetic radiation is directly absorbed by the sample, is preferably used because the temperature can be changed rapidly and directly in the sample without mechanical contact to the sample. Also preferred may be that said laser is a high-power laser within the range of from 0.01 W to 10 W, preferably from 4 W to 6 W. Also preferred may be that said laser is a laser within the range of from 1 mW to 1 W, preferably from 1 mW to 500 mW, more preferably from 1 mW to 250 mW. Laser radiation is directly absorbed by the sample and converted to heat, e.g., IR laser light of the wavelengths 980 nm +/- 30 nm, 1480 nm +/- 30 nm, 1550 nm +/- 30 nm, 1940 nm +/- 30 nm is very well absorbed by water and heats up very quickly. This heating method is contactless and may thus be fast and without the risk of contamination. The sample chamber must only be transparent to the laser light but does not require a good thermal conductivity, in contrast to contact heating by means of a heating element. With an IR laser, very small volumes ( e.g. in the nanoliter volume range) can be heated up, of which the fluorescence is measured by fluorescence optics (typically only 100 µm × 100 µm × 100 µm = 1 nl volume).

In another embodiment of the present invention, where said alterations in the fluorescence spectrum of said labelled protein comprising an Fc portion (e.g., antibody) is analysed by temperature-related intensity change, the temperature-related intensity change may comprise the following steps:
(a) providing a sample with said fluorescently labelled protein comprising an Fc portion (e.g., antibody) and particles in a solution;
(b) exciting the labelled protein comprising an Fc portion (e.g., antibody) and detecting the fluorescence of the excited protein comprising an Fc portion (e.g., antibody) at a predetermined temperature;
(c) repeating the two preceding steps multiple times at different concentrations of the particles; and
(d) determining a potential interaction between said labelled protein comprising an Fc portion (e.g., antibody) and said particles based on the particles concentration dependent change of the fluorescence of the labelled protein comprising an Fc portion (e.g., antibody).

In this context, where said alterations in the fluorescence spectrum of said labelled protein comprising an Fc portion (e.g., antibody) is analysed by temperature-related intensity change, step (b) may further comprise
(b1) exciting the labelled protein comprising an Fc portion (e.g., antibody) and detecting the fluorescence of the excited protein comprising an Fc portion (e.g., antibody) at a first predetermined temperature;
(b2) heating or cooling the solution to a second predetermined temperature; and
(b3) exciting the labelled protein comprising an Fc portion (e.g., antibody) and detecting the fluorescence of the excited protein comprising an Fc portion (e.g., antibody) at the second predetermined temperature.

In yet another embodiment of the present invention, where said alterations in the fluorescence spectrum of said labelled protein comprising an Fc portion (e.g., antibody) is analysed by spectral shift technology, the spectral shift technology may comprise the following steps:
(a) providing a sample with said fluorescently labelled protein comprising an Fc portion (e.g., antibody) and particles in a solution under first conditions,
(b) exciting said labelled protein comprising an Fc portion (e.g., antibody) at a first wavelength,
(c) detecting the fluorescence emission intensity of the labelled protein comprising an Fc portion (e.g., antibody) at a second and a third wavelength,
(d) calculating the ratio between said fluorescence intensities at the second and the third wavelength,
   wherein said third wavelength is different from said second wavelength;
   (e1) repeating steps (b) to (d) for said sample of the labelled protein comprising an Fc portion (e.g., antibody) under second conditions, or
   (e2) repeating steps (a) to (d) for a second sample of the labelled particles under second conditions,
      wherein said second conditions are different from said first conditions; and
(f) characterizing the labelled protein comprising an Fc portion (e.g., antibody) based on the calculated ratios obtained for the different conditions,
wherein the second and third wavelength are detected simultaneously, and wherein the second wavelength is shorter, and the third wavelength is longer than an emission maximum of the fluorescence emission of the labelled protein comprising an Fc portion (e.g., antibody) under the first conditions.

In context with the present invention, means for exciting may be an excitation light source, for example at least one light source from the group consisting of laser, laser fibre laser, diode laser, LED, HXP, Halogen, LED-Array, and HBO.

In a preferred embodiment of the present invention, where spectral shift technology is employed as described herein, a "dual-emission" configuration is used in combination with "red" fluorescent labels, such as Cy5, RFP, and other dyes known in the art and described herein. Since such labels have their excitation maximum at approx. 650 nm with a secondary excitation peak at approx. 600 nm and the emission maximum at approx. 660 nm, well-suited excitation and emission wavelengths include excitation between approx. 570 nm and 615 nm, detection of the first emission between approx. 625 nm and 650 nm and detection of the second emission between approx. 670 nm and 725 nm According to the present invention, the second wavelength is preferably detected at a shorter wavelength and the third wavelength is detected at a longer wavelength than an emission maximum of the fluorescently labeled particles under the first condition. The person skilled in the art is aware that in the context of the present invention an emission maximum can be a local emission maximum or an absolute emission maximum. Alternatively, the detection can be around a saddle point of the emission spectrum instead of an emission maximum. Small changes (e.g., wavelength shifts) in the regions flanking the emission maximum have a relatively large impact on the alterations of a fluorescence spectrum (e.g., in terms of intensities). Thus, in one embodiment of the present invention, the emission fluorescence intensities are detected in close proximity to an emission maximum, e.g., the second wavelength is detected at an at least 2.5 nm shorter wavelength (e.g., about 10 nm shorter wavelength) and the third wavelength is detected at an at least 2.5 nm longer wavelength (e.g., about 10 nm longer wavelength) than said emission maximum of the fluorescently labeled particles under first conditions.

According to the present invention, preferable means for detecting the excited fluorescently labeled Fc-binding peptides (or particles), particularly for detecting the fluorescence, may be any suitable device selected from the group consisting of charge coupled device (CCD) cameras (2D or line-scan CCD), Line-Cameras, Photomultiplier Tubes (PMT), silicon photomultipliers (siPMs), avalanche photodiodes (APD), photodiode arrays (PDAs), complementary metal-oxide-semiconductor (CMOS) cameras and the like.

In context with the present invention, means for detecting may be a light detector, for example at least one detector from the group consisting of PMT, siPM, APD, CCD, and CMOS camera.

In one embodiment of the present invention, the method may further comprise determining the concentration of said protein comprising an Fc portion (e.g., antibody) or thermal stability of said protein comprising an Fc portion (e.g., antibody).

The present invention also relates to the use of a kit for performing the method as described and provided herein in context with the present invention, comprising (i) a fluorescent label ready to be coupled to a Fc-binding peptide, and (ii) a Fc-binding peptide. In this context, for example, said fluorescent label may already be coupled to said Fc-binding peptide. Furthermore, in context with the use of a kit for performing the method as described and provided herein, said Fc-binding peptide may preferably be as defined herein in context with the method of the present invention. The kit preferably further comprises an instruction manual explaining the use of the kit in at least one of the methods of the present invention.

The present invention also relates to a kit for use in the method as described and provided herein in context with the present invention, comprising (i) a fluorescent label ready to be coupled to a Fc-binding peptide and (ii) a Fc-binding peptide. In this context, and in accordance with the present invention, said fluorescent label may already be coupled to said Fc-binding peptide. However, said fluorescent label may not already be coupled to said Fc-binding peptide. Furthermore, in context with the kit for use in performing the method as described and provided herein, said Fc-binding peptide may preferably be as defined herein in context with the method of the present invention. The kit preferably further comprises an instruction manual explaining the use of the kit in at least one of the methods of the present invention.

The present invention also relates to a system comprising a Fc-binding peptide comprising (i) a fluorescent label, (ii) a protein comprising an Fc portion (e.g., antibody) which is to be bound by said Fc-binding peptide and (iii) a device configured to analyse alterations in the fluorescence spectrum of said labelled protein comprising an Fc portion (e.g., antibody) upon a potential interaction of said labelled protein comprising an Fc portion (e.g., antibody) with particles. In this context, and in accordance with the present invention, said device may be configured to perform microscale thermophoresis, temperature-related intensity change or spectral shift measurement. For example, microscale thermophoresis, temperature-related intensity change or spectral shift measurement, respectively, may be carried out as described herein.

Said device configured to analyze alterations in the fluorescence spectrum of said labelled protein comprising an Fc portion (e.g., antibody) upon a potential interaction of said labelled protein comprising an Fc portion (e.g., antibody) with particles may be adapted for performing the methods described herein. In some aspects, particularly wherein said device is configured to perform spectral shift measurement, the device may comprise a sample holder for holding a sample of fluorescently labeled particles in solution under a plurality of conditions (i.e. multiple different conditions); means for exciting the fluorescently labeled particles at a first wavelength; means for detecting the fluorescence emission intensity of the fluorescently labeled particles at a second and a third wavelength; means for calculating a ratio between said fluorescence intensities at the second and the third wavelength, wherein said third wavelength is different from said second wavelength; wherein the device is configured to consecutively excite fluorescently, detect fluorescence emissions and calculate the ratio for samples at different conditions; and/ or means for characterizing the fluorescently labeled particles based on the calculated ratios obtained for the different conditions, wherein device is configured to simultaneously detect the second and third wavelength, and wherein the second wavelength is shorter and the third wavelength is longer than an emission maximum of the fluorescence emission of the fluorescently labeled particles under a first condition (of the different conditions).

The present invention also relates to the use of the system as described and provided herein for measuring an interaction between a fluorescently labelled protein comprising an Fc portion (e.g., antibody) and particles in a solution. In this context, said measuring of an interaction between a fluorescently labelled protein comprising an Fc portion (e.g., antibody) and particles in a solution may be pursuant to the method as described and provided in accordance with the present invention.

The embodiments which characterize the present invention are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% or 2% of a given value or range, and also comprise the respective exact numeric value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The Figures show:
- **Figure 1**: FcT vs Trastuzumab SpS
- **Figure 2**: Fclll vs Trastuzumab SpS
- **Figure 3**: FcT vs Trastuzumab TRIC signal at 670 nm
- **Figure 4**: Fclll vs Trastuzumab TRIC signal at 670 nm
- **Figure 5**: Interaction of Her2 with Trastuzumab labeled with FcT in SpS
- **Figure 6**: Interaction of Her2 with Trastuzumab labeled with FcT in TRIC 670 nm
- **Figure 7**: Interaction of Her2 with Trastuzumab labeled with Fclll
- **Figure 8**: Interaction of Her2 with Trastuzumab labeled with Fclll in TRIC 670 nm
- **Figure 9**: Interaction of B.1.1.7. with CR3022 labeled with FcT in SpS
- **Figure 10**: Interaction of B.1.1.7. with CR3022 labeled with Fclll in SpS
- **Figure 11**: Interaction of Her2 with Trastuzumab labeled via NHS in SpS
- **Figure 12**: Interaction of Her2 with Trastuzumab labeled via NHS in TRIC 670 nm
- **Figure 13**: Thermal unfolding of Trastuzumab labeled with Fclll; 13A: 670 / 650 nm ratio; 13B: first derivative
- **Figure 14**: Thermal unfolding of FcIII; 14A: 670 / 650 nm ratio; 14B: first derivative

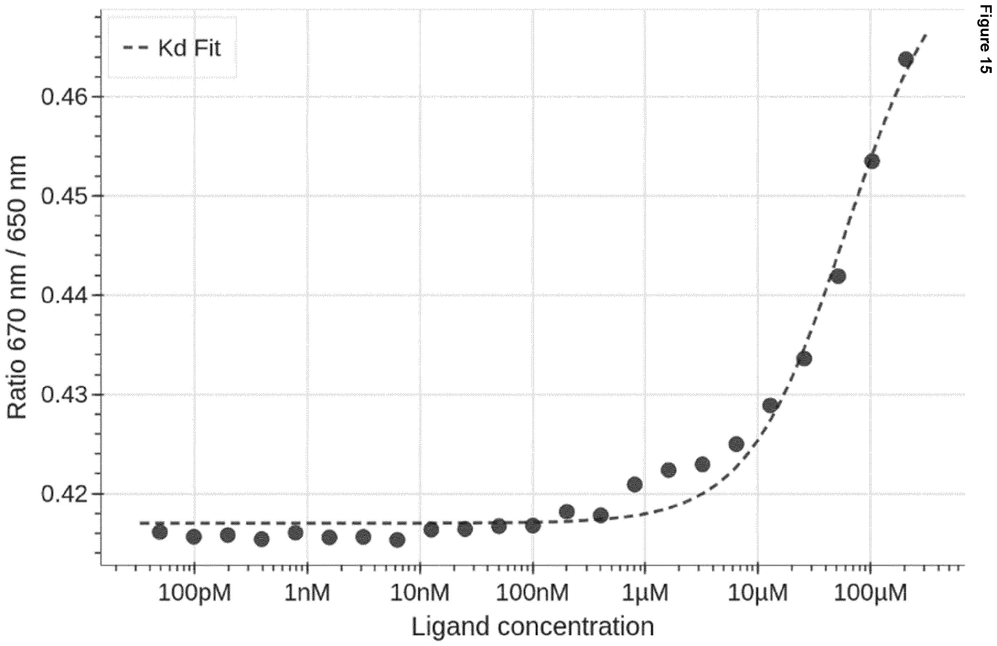

- **Figure 15**: Ratiometric change in fluorescence at 670/650 nm upon binding of Cy5-KAPAR (cf. SEQ ID NO: 9) to Trastuzumab

The following Sequences are provided herein:
SEQ ID NO: 1
   Fc-III/ doi:10.1126/science.287.5456.1279
   protein
   artificial
   DCAWHLGELVWCT
SEQ ID NO: 2
   FcBP2/ doi:10.1021/ja057513w
   protein
   artificial
   PDCAWHLGELVWCTP
SEQ ID NO: 3
   Fc-III-4C/ doi:10.1021/acs.bioconjchem.6b00170
   protein
   artificial
   CDCAWH LGELVWCTC
SEQ ID NO: 4
   doi:10.1021 /ac4029467
   protein
   artificial
   RRGW
SEQ ID NO: 5
   doi:10.1007/s13206-016-0202-z
   protein
   artificial
   KHRFNKD
SEQ ID NO: 6
   doi:10.1074/jbc. M 112.389742
   protein
   artificial
   HMVCLAYRGRPVCFAL
SEQ ID NO: 7
   doi:10.1074/jbc. M 112.389742
   protein
   artificial
   HMVCLSYRGRPVCFSL
SEQ ID NO: 8
   doi:10.1074/jbc. M 112.389742
   protein
   artificial
   HQVCLSYRGRPVCFST
SEQ ID NO: 9
   protein
   artificial
   KAPAR

The invention is further illustrated by the following examples, however, without being limited to the example or by any specific embodiment of the examples.

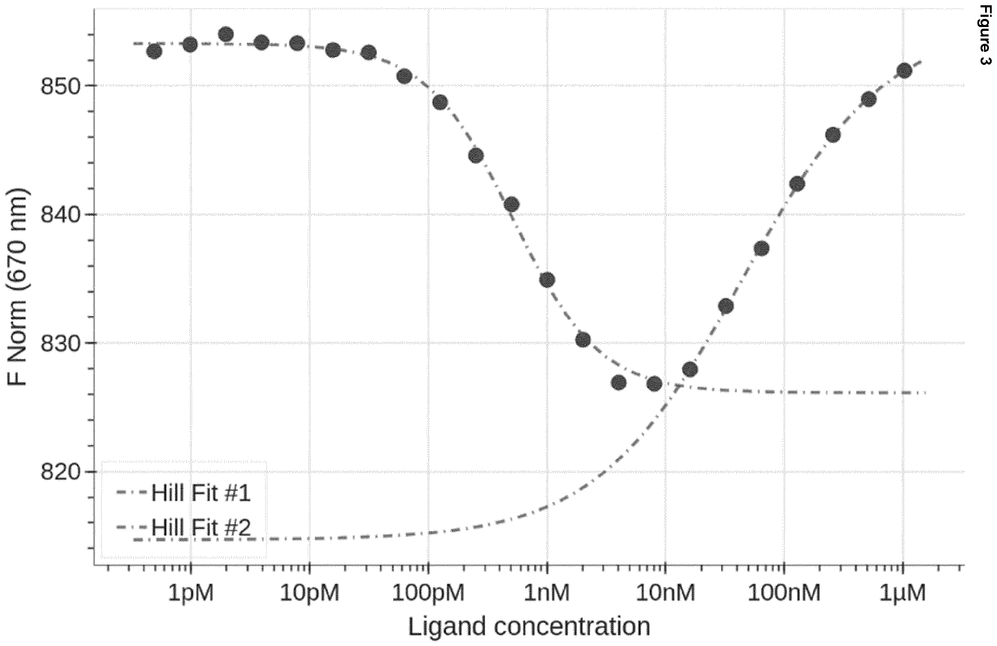

The present invention can also be characterized by the following items:
1. A method for measuring an interaction between a fluorescently labelled protein comprising an Fc portion and one or more particles in a solution, comprising analysing alterations in the fluorescence spectrum of said labelled protein comprising an Fc portion upon a potential interaction with said particles, wherein said protein comprising an Fc portion is fluorescently labelled by a Fc-binding peptide capable of binding to said protein comprising an Fc portion within its Fc region, said Fc-binding peptide comprising a fluorescent label.
2. The method of any one of the preceding items, wherein alterations in the fluorescence spectrum are alterations in the fluorescence emission spectrum.
3. The method of any one of the preceding items, wherein the labelled protein comprising an Fc portion is dissolved or dispersed in the solution or is immobilized on a solid support.
4. The method of any one of the preceding items, wherein said particles are dissolved or dispersed in the solution or is immobilized on a solid support.
5. The method of any one of the preceding items, wherein said solution further comprises a compound which may suppress, inhibit or enhance an interaction between said labelled protein comprising an Fc portion and said particles.
6. The method of any one of the preceding items, wherein an alteration in the fluorescence spectrum of said labelled protein comprising an Fc portion is indicative of an interaction between said labelled protein comprising an Fc portion and said particles.
7. The method of any one of the preceding items, wherein as a control the fluorescence spectrum of said labelled protein comprising an Fc portion is analysed in the absence of said particles.
8. The method of any one of the preceding items, wherein said particles are selected from the group consisting of organic molecules, biomolecules, nanoparticles, microparticles, vesicles, biological cells or sub-cellular fragments, biological tissues, viral particles, viruses, cellular organelles, lipid nanoparticles (LNPs), and virus like particles.
9. The method of item 8, wherein said biomolecules are selected from the group consisting of amino acids, proteins, peptides, mono- and disaccharides, polysaccharides, lipids, glycolipids, fatty acids, sterols, vitamins, neurotransmitter, enzymes, nucleotides, metabolites, nucleic acids, and combinations thereof.
10. The method of any one of the preceding items, wherein said interaction is binding between said protein comprising an Fc portion and said particles.
11. The method of any one of the preceding items, wherein said particles comprise a protein comprising an epitope for said labelled protein comprising an Fc portion.
12. The method of item 11, wherein said labelled protein comprising an Fc portion interacts with said epitope.
13. The method of any one of the preceding items, wherein said particles comprise the same protein comprising an Fc portion as the fluorescently labelled protein comprising an Fc portion, with the exception that it does not comprise the same fluorescent label as said fluorescently labelled protein comprising an Fc portion.
14. The method of any one of the preceding items, wherein said Fc-binding peptide is non-covalently bound to said protein comprising an Fc portion.
15. The method of any one of the preceding items, wherein said Fc-binding peptide remains bound to said protein comprising an Fc portion up to a temperature of at least about 60 °C.
16. The method of any one of the preceding items, wherein said Fc-binding peptide has an affinity to said protein comprising an Fc portion of at least about 1×10⁻⁷ M.
17. The method of any one of the preceding items, wherein said Fc-binding peptide comprises 4 to 30 amino acids.
18. The method of any one of the preceding items, wherein said Fc-binding peptide comprises at least one linker.
19. The method of item 18, wherein said linker comprised by said Fc-binding peptide is activatable.
20. The method of item 18 or 19, wherein said linker comprised by said Fc-binding peptide comprises at least one moiety which allows covalent binding with an amino acid or peptide within the Fc region of said protein comprising an Fc portion upon binding of said Fc-binding peptide to said protein comprising an Fc portion.
21. The method of item 20, wherein said Fc-binding peptide is covalently bound to said protein comprising an Fc portion.
22. The method of any one of the preceding items, wherein said protein comprising an Fc portion is an antibody.
23. The method of item 22, wherein said antibody is an IgG antibody.
24. The method of any one of the preceding items, wherein said Fc-binding peptide is
   (a) DCAWHLGELVWCT (SEQ ID NO: 1),
   (b) L-Pro DCAWHLGELVWCT D-Pro (SEQ ID NO: 2),
   (c) CDCAWHLGELVWCTC (SEQ ID NO: 3),
   (d) RRGW (SEQ ID NO: 4),
   (e) KHRFNKD (SEQ ID NO: 5), or
   (f) or any one of Fc-binding peptides as shown in Table 2 of doi: 10.3390/ma9120994
   (g) or any one of Fc-binding peptides described in the "Abstract" of 10.1016/j.bej.2013.06.017
   (h) or the Fc-binding peptide shown in Figure 3a of 10.1021/acs.bioconjchem.8b00865
25. The method of item 22, wherein said antibody is an IgA antibody.
26. The method of item 25, wherein said Fc-binding peptide is
   (a) HMVCLAYRGRPVCFAL (SEQ ID NO: 6),
   (b) HMVCLSYRGRPVCFSL (SEQ ID NO: 7), or
   (c) HQVCLSYRGRPVCFST (SEQ ID NO: 8).
27. The method of any one of the preceding items, wherein the fluorescent label is a fluorescent dye.
28. The method of item 27, wherein said fluorescent dye is Cy5, Cy3, Atto647, Atto647N, Alexa647, Dy647, or variants thereof.
29. The method of any one of the preceding items, wherein the alterations in the fluorescence spectrum of said labelled protein comprising an Fc portion are analysed by microscale thermophoresis, temperature-related intensity change or spectral shift technology.
30. The method of item 29, wherein microscale thermophoresis comprises the following steps:
   (a) providing a sample with said fluorescently labelled protein comprising an Fc portion and particles in a solution;
   (b) exciting said fluorescently labelled protein comprising an Fc portion and firstly detecting fluorescence of said excited protein comprising an Fc portion;
   (c) irradiating a laser light beam into the solution to obtain a spatial temperature distribution in the solution around the irradiated laser light beam;
   (d) detecting secondly a fluorescence of the labelled protein comprising an Fc portion in the solution at a predetermined time after irradiation of the laser into the solution has been started, and
   (e) determining a potential interaction between said labelled protein comprising an Fc portion and said particles based on said first and second detection.
31. The method of item 30, wherein the irradiation of the laser and the detection of the fluorescence is conducted from the same side with respect to the sample.
32. The method of item 29, wherein said temperature-related intensity change comprises the following steps:
   (a) providing a sample with said fluorescently labelled protein comprising an Fc portion and particles in a solution;
   (b) exciting the labelled protein comprising an Fc portion and detecting the fluorescence of the excited protein comprising an Fc portion at a predetermined temperature;
   (c) repeating the two preceding steps multiple times at different concentrations of the particles; and
   (d) determining a potential interaction between said labelled protein comprising an Fc portion and said particles based on the particles concentration dependent change of the fluorescence of the labelled protein comprising an Fc portion.
33. The method of item 32, wherein step (b) comprises
   (b1) exciting the labelled protein comprising an Fc portion and detecting the fluorescence of the excited protein comprising an Fc portion at a first predetermined temperature;
   (b2) heating or cooling the solution to a second predetermined temperature;
   (b3) exciting the labelled protein comprising an Fc portion and detecting the fluorescence of the excited protein comprising an Fc portion at the second predetermined temperature.
34. The method of item 29, wherein said spectral shift technology comprises the following steps:
   (a) providing a sample with said fluorescently labelled protein comprising an Fc portion and particles in a solution under first conditions,
   (b) exciting said labelled protein comprising an Fc portion at a first wavelength,
   (c) detecting the fluorescence emission intensity of the labelled protein comprising an Fc portion at a second and a third wavelength,
   (d) calculating the ratio between said fluorescence intensities at the second and the third wavelength, wherein said third wavelength is different from said second wavelength;
   (e1) repeating steps (b) to (d) for said sample of the labelled protein comprising an Fc portion under second conditions, or
   (e2) repeating steps (a) to (d) for a second sample of the labelled particles under second conditions, wherein said second conditions are different from said first conditions;
   (f) characterizing the labelled protein comprising an Fc portion based on the calculated ratios obtained for the different conditions,
   wherein the second and third wavelength are detected simultaneously, and wherein the second wavelength is shorter, and the third wavelength is longer than an emission maximum of the fluorescence emission of the labelled protein comprising an Fc portion under the first conditions.
35. The method of any one of the preceding items, further comprising determining the concentration of said protein comprising an Fc portion or thermal stability of said protein comprising an Fc portion.
36. Use of a kit for performing the method of any one of items 1 to 35, comprising (i) a fluorescent label ready to be coupled to a Fc-binding peptide and (ii) a Fc-binding peptide.
37. The use of item 36, wherein said fluorescent label is already coupled to said Fc-binding peptide.
38. The use of item 36 or 37, wherein said Fc-binding peptide is defined in any one of items 14 to 22, 24 and 26.
39. A kit for use in the method of any one of items 1 to 35, comprising (i) a fluorescent label ready to be coupled to a Fc-binding peptide and (ii) a Fc-binding peptide.
40. The kit for the use of item 39, wherein said fluorescent label is already coupled to said Fc-binding peptide.
41. The kit for the use of item 39 or 40, wherein said Fc-binding peptide is defined in any one of items 14 to 22, 24 and 26.
42. A system comprising a Fc-binding peptide comprising (i) a fluorescent label, (ii) a protein comprising an Fc portion which is to be bound by said Fc-binding peptide and (iii) a device configured to analyse alterations in the fluorescence spectrum of said labelled protein comprising an Fc portion upon a potential interaction of said labelled protein comprising an Fc portion with particles.
43. The system of item 42, wherein said device is configured to perform microscale thermophoresis, temperature-related intensity change or spectral shift measurement.
44. The system of item 43, wherein microscale thermophoresis is defined in any one of items 29 to 31, temperature-related intensity change is defined in any one of items 29, 32 or 33, and spectral shift technology is defined in item 29 or 34.
45. Use of the system of any one of items 42 to 44 for measuring an interaction between a fluorescently labelled protein comprising an Fc portion and particles in a solution.
46. The use of item 45, wherein said measuring of an interaction between a fluorescently labelled protein comprising an Fc portion and particles in a solution is pursuant to the method of any one of items 1 to 35.
47. The system of any one of items 42 to 44, or the use of items 45 or 46, wherein said protein comprising an Fc portion is an antibody.

### Examples

### Materials

- Fclll: [Cyc(2,12)]Cy5-DCAWHLGELVWCT-NH₂ (cf. SEQ ID NO: 1) // Purity >97 % synthesized by Biosynth
- FcT: [Cyc(2,12)]NTTred-DCAWHLGELVWCT-OH (cf. SEQ ID NO: 1) // Purity >90 % synthesized by Biosynth
- Cy5-KAPAR: Cy5-KAPAR-OH (cf. SEQ ID NO: 9) // Purity 100 % synthesized by Biosynth
- SARS-CoV-2 Spike Protein (B.1.1.7 - alpha) // antibodies-online.com CR3022: Recombinant SARS-CoV-2 Spike S1 antibody (RBD) // antibodies-online.com
- Trastuzumab
- Her2/ERBB2 Protein, Human, Recombinant (ECD, His Tag), # 10004-H08H, Sino Biological
- PBS-P: Phosphate buffered saline, Pluronic F 0.05%
- DMSO (100%)

### Methods and results

The following example describes the use of the fluorescently labeled peptide tracer according to the present invention in order to obtain a dose-response curve between a native Trastuzumab antibody and said peptides.

The fluorescent peptide tracer was dissolved in 100% DMSO to obtain stock solutions of 2.4 mM for Fclll and FcT. Subsequently, 16 or 24 point 1-to-1 dilution series of unlabeled Trastuzumab antibodies with stock concentrations of 825 µM were prepared leading to final assay concentrations of 2062.5 nM for Trastuzumab and a FcT and Fclll concentration of 2.4 nM. Samples were loaded into Dianthus 384-well plates (DI-P001A, NanoTemper Technologies) or polymer-coated borosilicate glass capillaries (Monolith NT.115 Premium Capillaries, MO-K025, NanoTemper Technologies). Measurements were taken with NanoTemper Software (MO.Control 2, DI.Control) by excitation at 591 nm and recording of fluorescence traces at 650 nm and 670 nm. For results showing TRIC results, simultaneous induction heat via an IR laser was induced and the thermophoretic behavior recorded for 5s.

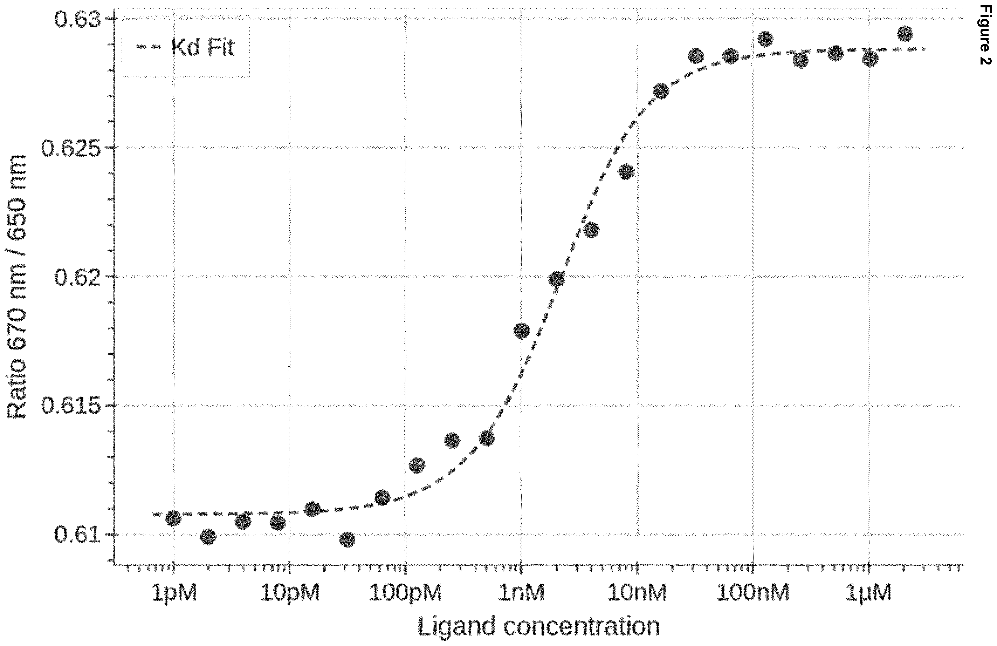

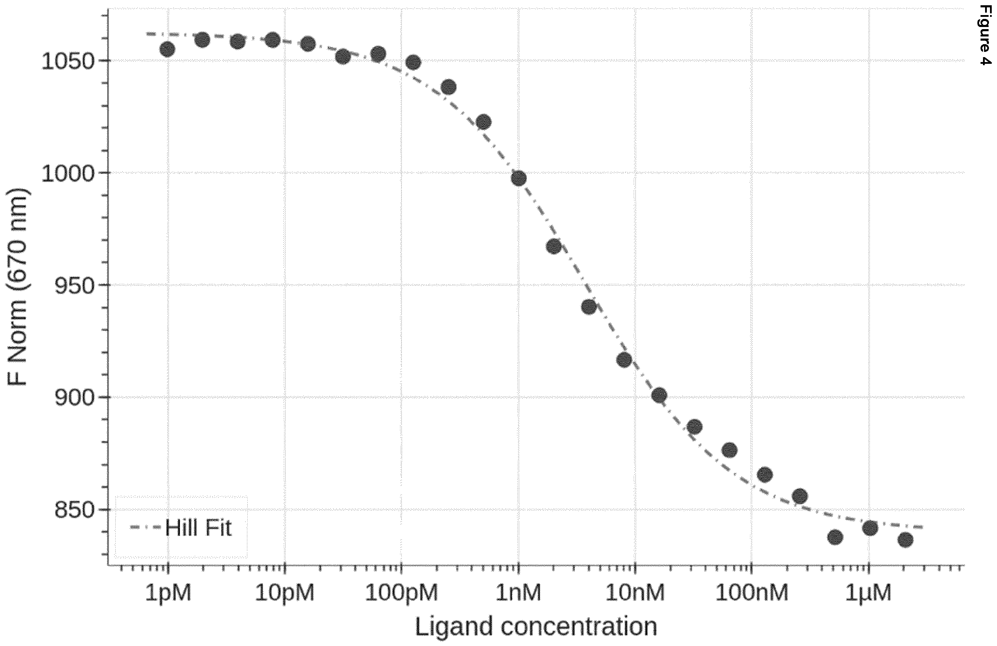

Clear binding with nanomolar affinities of the fluorescent peptides to the antibody can be seen in Fig 1 and 2 using spectral shift response (SpS), but also TRIC (Fig 3 and 4) for FcT and Fclll, respectively.

### Interaction of Her2 with Trastuzumab labeled via Fclll and FcT in spectral shift:

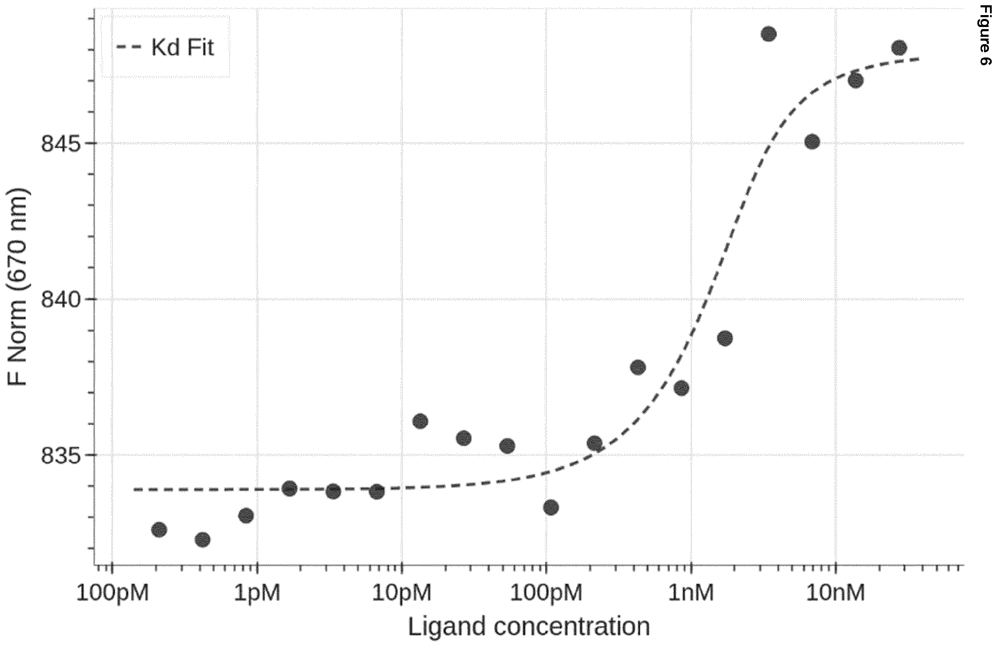

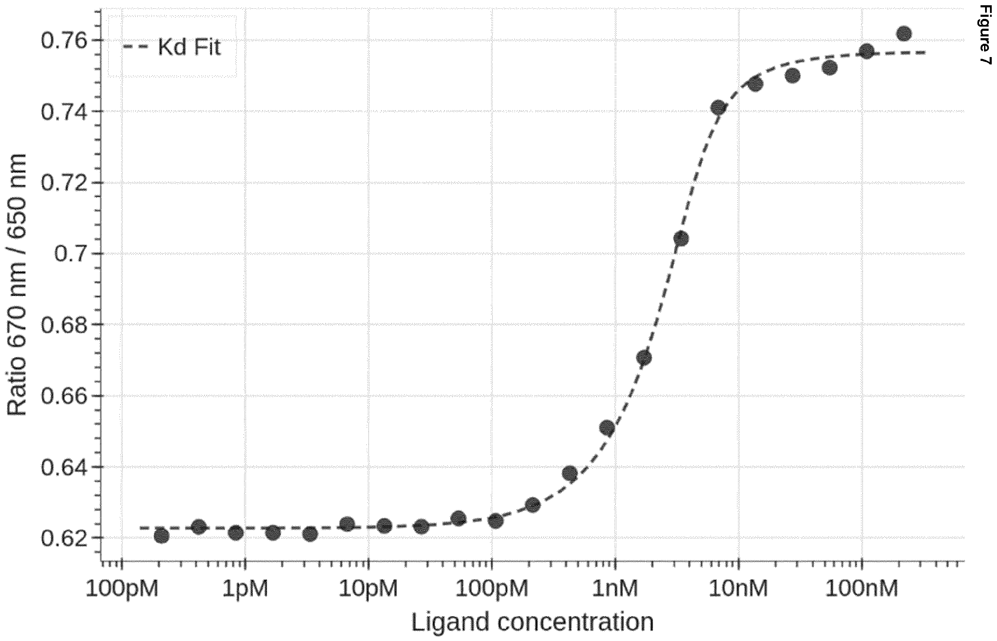

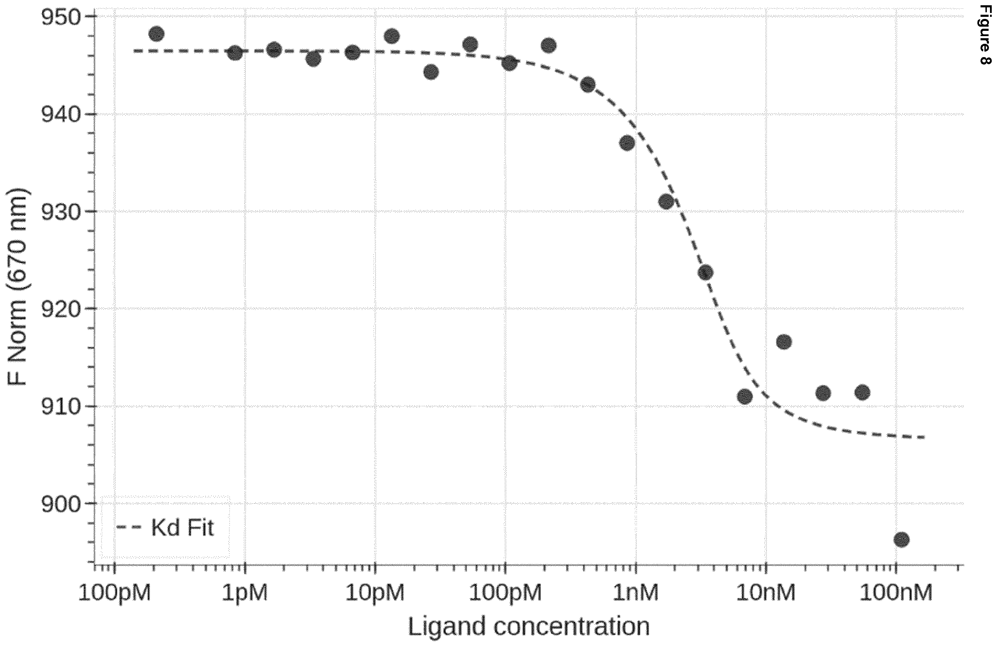

The fluorescent peptide tracer was dissolved in 100% DMSO to obtain stock solutions of 2.4 mM for Fclll and FcT. Peptide tracers were mixed with 8 nM Trastuzumab and incubated for 15 min. Subsequently, 16 or 24 point 1-to-1 dilution series of Her2 were prepared and mixed 1:1 with prepared peptide:trastuzumab dilution, leading to final assay concentrations of 4 nM for Trastuzumab, 2.4 nM FcT and Fclll, and 220 nM as highest in-assay concentration of Her2. Samples were loaded into Dianthus 384-well plates (DI-P001A, NanoTemper Technologies) or polymer-coated borosilicate glass capillaries (Monolith NT.115 Premium Capillaries, MO-K025, NanoTemper Technologies). Measurements were taken with NanoTemper Software (MO.Control 2, DI.Control) by excitation at 591 nm and recording of fluorescence traces at 650 nm and 670 nm. For results showing TRIC results, simultaneous heat via an IR laser was induced and the thermophoretic behavior recorded for 5s. The resulting dose-response curves clearly show the binding of Her2 in spectral shift (Fig. 5 and 7) and TRIC signal (Fig. 6 and 8) for FcT and Fclll, respectively.

### Interaction of B.1.1.7 with CR3022 labeled via Fclll and FcT in spectral shift:

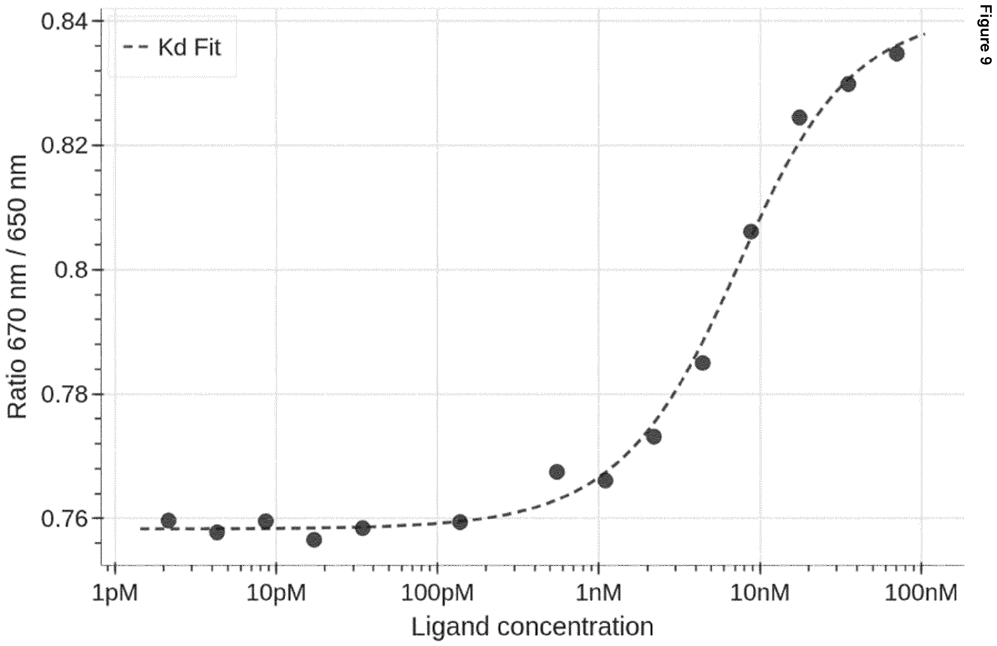

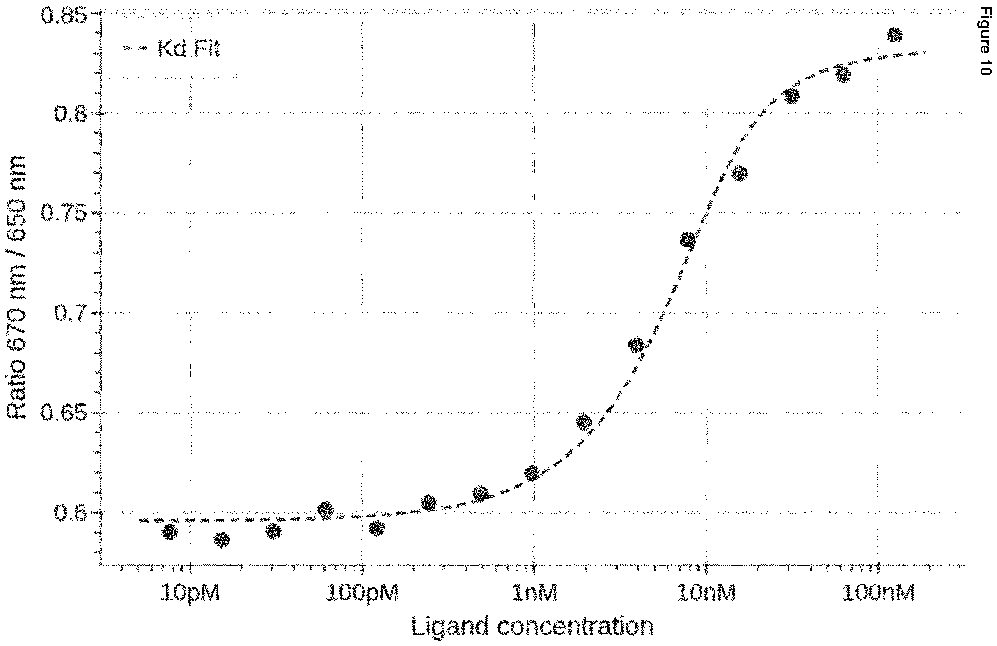

The fluorescent peptide tracer was dissolved in 100% DMSO to obtain stock solutions of 2.4 mM for Fclll and FcT. Subsequently, 16 point 1-to-1 dilution series of - SARS-CoV-2 Spike Protein (B.1.1.7) were prepared leading to final assay concentrations of 10 nM for CR3022 and a FcT and Fclll concentration of 5 nM. Samples were loaded into Dianthus 384-well plates (DI-P001A, NanoTemper Technologies) or polymer-coated borosilicate glass capillaries (Monolith NT.115 Premium Capillaries, MO-K025, NanoTemper Technologies). Measurements were taken with NanoTemper Software (MO.Control 2, DI.Control) by excitation at 591 nm and recording of fluorescence traces at 650 nm and 670 nm. Results for FcT and Fclll, respectively, are shown in Fig. 9 and 10.

### Comparison with NHS-labeled Trastuzumab:

For NHS labeling of Trastuzumab, NHS labeling kit 2^{nd} generation (NanoTemper Technologies) was used. In short, Trastuzumab was exchanged into NHS labeling buffer and mixed with NHS-NTT dye. After incubation in the dark for 30 min, unreacted dye was separated using a B-column provided with the kit. Eluting fractions containing 2.5 µM labeled Trastuzumab were collected and stored at -80 °C. A 1:1 serial dilution of Her2 was performed using 1408 nM Her2 and 5 nM NHS-labeled Trastuzumab were added, leading to final assay concentrations of 2.5 nM Trastuzumab and 704 nM highest in-assay concentration of Her2. A dose-response can be seen in the 670/650 nm ratio (Fig. 11) but not in TRIC (Fig. 12).

### Using Fc-peptide labeling for thermal unfolding analysis:

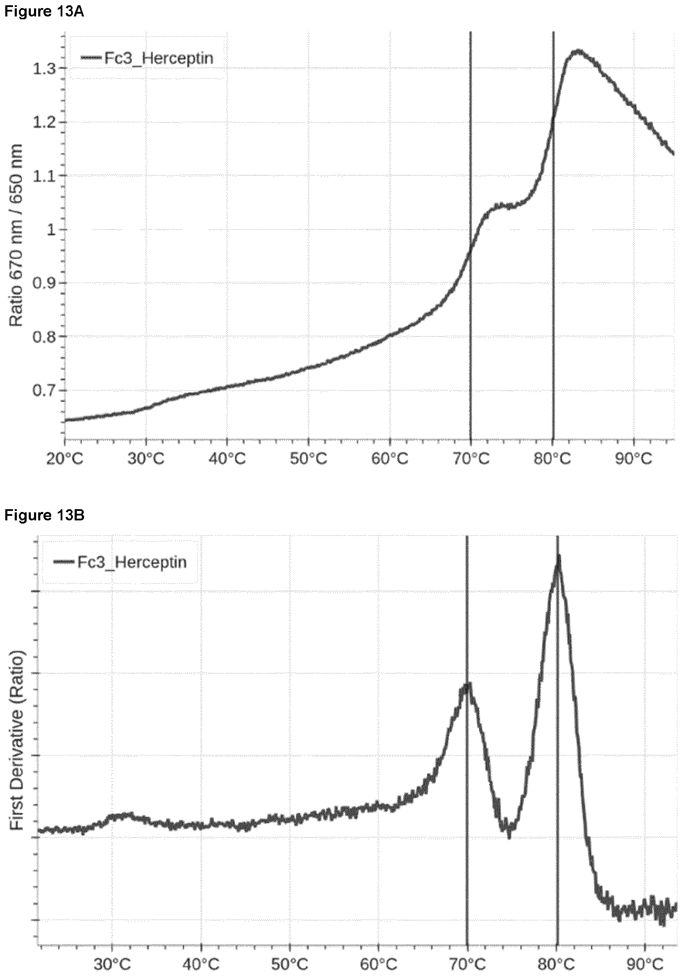

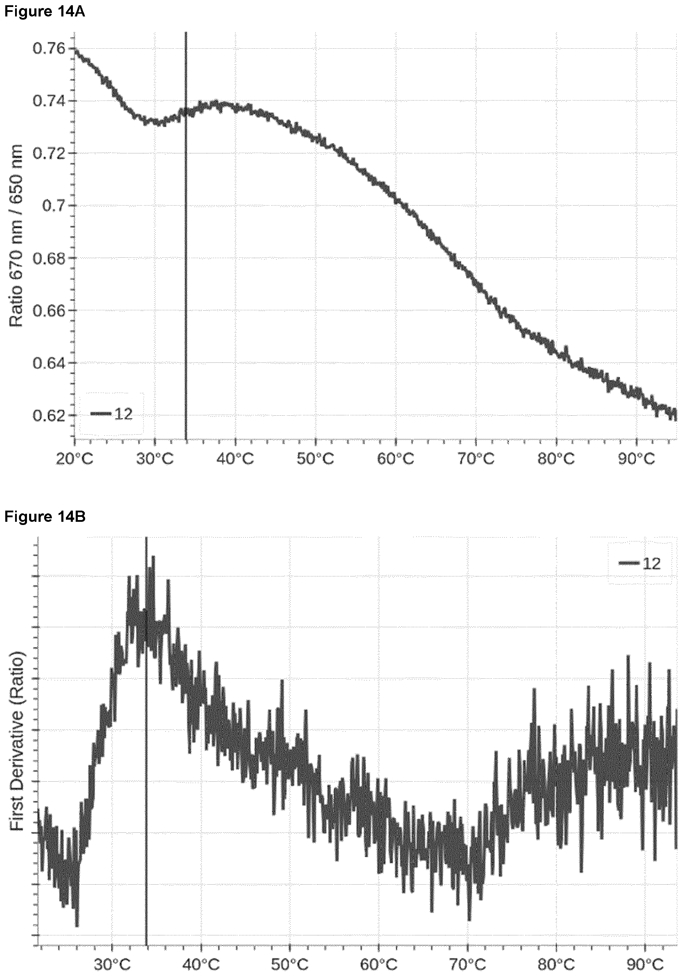

The fluorescent peptide tracer was dissolved in 100% DMSO to obtain stock solutions of 2.4 mM for Fclll. The stock solution was further diluted in PBS-P and mixed with Trastuzumab resulting in final assay concentrations of 120 nM for Fclll and 1.4 µM for Trastuzumab. Samples were loaded into capillaries, excited at 591 nm and fluorescence was recorded at 650 and 670 nm. Samples were heated a rate of 1 °C /min while recording fluorescence signals. The resulting thermal profile of free Fclll shown in Fig. 14A shows a slow decrease in the 670 / 650 nm ratio with no distinct unfolding events visible in the first derivative (Fig. 14B). In contrast, Trastuzumab labeled with Fclll shows a resulting unfolding profile with two distinct unfolding events at 70 °C and 80 °C corresponding to unfolding of the Fab and Fab + Fc part of the antibody (see Fig. 13A, 13B), comparable with literature data. This method of labeling allows a reliable and sensitive method for determination of antibody unfolding using a convenient mix and measure approach.

### Low affinity peptides for Fc-labeling:

To evaluate the importance of the right affinity for this labeling approach, also a fluorescent peptide tracer was designed based on previously published findings based on a combinatorial tetrapeptide library (Camperi et al., Biotechnol Lett (2003), 25: 1545-1548). To this end, the tetrapeptide APAR, which was previously found to bind to Immunoglobulin G was synthesized with an N-terminal lysine to accommodate further conjugation with Cy5 dye. The resulting fluorescent peptide tracer Cy5-KAPAR (cf. SEQ ID NO: 9) was dissolved in 100% DMSO to obtain stock solutions of 4.96 mM. The stock solution was further diluted in PBS-P. Subsequently 24 point 1-to-1 dilution series of unlabeled Trastuzumab antibody with stock concentrations of 825 µM were prepared leading to final assay concentrations of 412 µM for Trastuzumab and a Cy5-KAPAR (cf. SEQ ID NO: 9) concentration of 2.5 nM. Samples were loaded into Dianthus 384-well plates (DI-P001A, NanoTemper Technologies). Measurements were taken with NanoTemper Software (DI.Control) by excitation at 591 nm and recording of fluorescence traces at 650 nm and 670 nm. Fig. 14A shows the ratiometric change in the 670/650 nm fluorescence. It can clearly be seen that a large ratiometric change occurs upon binding of the Trastuzumab antibody. However, affinities are as expected significantly lower and are estimated to be above 60 µM as saturation could not be reached. Such affinities result in required assay concentrations of over 100 µM in ligand-binding assays, which can hardly be achieved due to antibody solubility and availability. Furthermore, only affinities lower than the used concentration of antibody could reliable be determined, severely limiting the approach.

## Claims

1. A method for measuring an interaction between a fluorescently labelled protein comprising an Fc portion and one or more particles in a solution, comprising analysing alterations in the fluorescence spectrum of said labelled protein comprising an Fc portion upon a potential interaction with said particles, wherein said protein comprising an Fc portion is fluorescently labelled by a Fc-binding peptide capable of binding to said protein comprising an Fc portion within its Fc region, said Fc-binding peptide comprising a fluorescent label, wherein the alterations in the fluorescence spectrum of said labelled protein comprising an Fc portion are analysed by microscale thermophoresis, temperature-related intensity change or spectral shift technology.

2. The method of claim 1, wherein alterations in the fluorescence spectrum are alterations in the fluorescence emission spectrum.

3. The method of any one of the preceding claims, wherein an alteration in the fluorescence spectrum of said labelled protein comprising an Fc portion is indicative of an interaction between said labelled protein comprising an Fc portion and said particles.

4. The method of any one of the preceding claims, wherein said particles are selected from the group consisting of organic molecules, biomolecules, nanoparticles, microparticles, vesicles, biological cells or sub-cellular fragments, biological tissues, viral particles, viruses, cellular organelles, lipid nanoparticles (LNPs), and virus like particles.

5. The method of any one of the preceding claims, wherein said Fc-binding peptide is non-covalently bound to said protein comprising an Fc portion.

6. The method of any one of the preceding claims, wherein said Fc-binding peptide has an affinity to said protein comprising an Fc portion of at least about 1×10⁻⁷ M.

7. The method of any one of the preceding claims, wherein said Fc-binding peptide comprises 4 to 30 amino acids.

8. The method of any one of the preceding claims, wherein said protein comprising an Fc portion is an antibody.

9. The method of any one of the preceding claims, wherein said Fc-binding peptide is
(a) DCAWHLGELVWCT (SEQ ID NO: 1),
(b) L-Pro DCAWHLGELVWCT D-Pro (SEQ ID NO: 2),
(c) CDCAWHLGELVWCTC (SEQ ID NO: 3),
(d) RRGW (SEQ ID NO: 4),
(e) KHRFNKD (SEQ ID NO: 5), or
(f) HMVCLAYRGRPVCFAL (SEQ ID NO: 6),
(g) HMVCLSYRGRPVCFSL (SEQ ID NO: 7), or
(h) HQVCLSYRGRPVCFST (SEQ ID NO: 8).

10. The method of any one of the preceding claims, wherein the fluorescent label is a fluorescent dye.

11. Use of a kit for performing the method of any one of claims 1 to 10, comprising (i) a fluorescent label ready to be coupled to a Fc-binding peptide and (ii) a Fc-binding peptide.

12. A kit for use in the method of any one of claims 1 to 10, comprising (i) a fluorescent label ready to be coupled to a Fc-binding peptide and (ii) a Fc-binding peptide.

13. The use of a kit according to claim 11 or the kit for use according to claim 12, wherein said fluorescence label is already coupled to said Fc-binding peptide.

14. A system comprising a Fc-binding peptide comprising (i) a fluorescent label, (ii) a protein comprising an Fc portion which is to be bound by said Fc-binding peptide and (iii) a device configured to analyse alterations in the fluorescence spectrum of said labelled protein comprising an Fc portion upon a potential interaction of said labelled protein comprising an Fc portion with particles, wherein said device is configured to perform microscale thermophoresis, temperature-related intensity change or spectral shift measurement.

15. Use of the system of claim 14 for measuring an interaction between a fluorescently labelled protein comprising an Fc portion and particles in a solution.
